# EUROPEAN PATENT SPECIFICATION

(11) **EP 3 889 268 B1**
(45) Date of publication and mention of the grant of the patent: **11.09.2024**
(21) Application number: 21165591.5
(22) Date of filing: 29.03.2021
(51) Int. Cl.: C12Q 1/04, G01N 33/487, G01N 27/00

(54) **METHOD FOR PATHOGEN DETECTION**
VERFAHREN ZUR ENTDECKUNG VON KRANKHEITSERREGERN
PROCÉDÉS DE DÉTECTION DE PATHOGÈNES

(30) Priority: 30.03.2020 US 202063001938 P
(43) Date of publication of application: 06.10.2021
(73) Proprietor: Helios Bioelectronics Inc., Zhubei City, 30261 (TW)
(72) Inventor: CHAN, Hardy Wai Hong, 241 NEW TAIPEI CITY (TW); LIU, Yi-Shao, 302 ZHUBEI CITY (TW); CHEN, Chihchung, 302 ZHUBEI CITY (TW); LIN, Ming-Yu, 310 ZHUDONG TOWNSHIP (TW); LEE, Hao, 970 HUALIEN CITY (TW)
(74) Representative: Fuchs Patentanwälte Partnerschaft mbB

(56) References cited:
- EP-A1- 0 313 411
- WO-A1-2013/096404
- WO-A2-2008/042003
- GB-A- 2 131 954

## Description

### CROSS REFERENCE TO RELATED APPLICATIONS

This application claims the benefit of prior-filed provisional application with application serial number 63/001,938, filed on March 30, 2020.

### FIELD OF THE INVENTION

The present invention relates to bio-sample detection and therapy suggestion, more particularly to a method for pathogen detection and associated therapy development and suggestions for physicians.

### BACKGROUND OF THE INVENTION

Identification of microorganisms is clinically critical. The conventional procedure of microorganism identification is culturing a sample with medium for several days and observing the morphology as well as the compositions of the microorganism and optionally performing more tests. Moreover, drug sensitivity and effective dosage assays play important roles in treating infection. The conventional procedure of drug sensitivity assay is applying a candidate agent to microorganism cultures and observing if any growth suppression appears, which even takes weeks to obtain the result. Currently, spectroscopy is utilized for assisting the microorganism growth observation based on turbidity of the culturing medium. Due to the sensitivity of the spectrum, the signal can only be detected when the count of microorganism reaches 10⁸ cfu/mL, usually after 16 to 48 hours of culturing. In another aspect, polymerase chain reaction and MALDI-TOF are also applied in accurate microorganism identification. However, such techniques cannot be used in drug sensitivity assay.

The patent application GB2131954 describes an electrochemical method for detecting and classifying microbial cells comprising the steps of: applying a sweep potential between a working electrode and a counter electrode while the microbial cells are brought into contact with the working electrode and, then, measuring the generated current between the electrodes, wherein the cell numbers are determined by measuring the peak current of the generated current.

The current procedures of pathogen identification and drug sensitivity assay are time consuming and limited.

### SUMMARY OF THE INVENTION

The claimed invention in its broadest aspect concerns a method for pathogen detection, comprising: applying a biological sample to a culturing chamber comprising an interacting agent, wherein the interacting agent is an agent that interacts with a pathogen; driving a sensing chip in direct contact with a culturing medium containing the interacting agent in the culturing chamber, wherein the culturing medium comprises the biological sample; measuring an electrical signal from the sensing chip by measuring a drain current of a transistor over a predetermined period, wherein the predetermined period is less than 6 hours; obtaining pathogen-related information of the biological sample based on the electrical signal; and determining the existence of at least a blip in the drain current during the predetermined period, wherein the blip is a statistically distinguishable signal in the drain current as in independent claim 1. Preferred embodiments are defined in dependent claims 2-12.

### SUMMARY OF THE DISCLOSURE

In one aspect, the present disclosure provides a method for pathogen detection, including operations that applying a biological sample to a culturing chamber comprising an interacting agent; driving a sensor electrically coupled to the biological sample in the culturing chamber; measuring an electrical signal from the sensor; and obtaining pathogen-related information of the biological sample based on the electrical signal.

In one embodiment of the disclosure, measuring the electrical signal from the sensor includes measuring a drain current of a transistor over a predetermined period.

In one embodiment of the disclosure, obtaining pathogen-related information of the biological sample includes determining the existence of at least a blip in the drain current during the predetermined period.

In one embodiment of the disclosure, the predetermined period is less than about 12 hours.

In one embodiment of the disclosure, the applying the biological sample to a plurality of culturing chambers includes applying the biological sample containing less than 100 colony-forming unit (CFU) per 100 µL.

In one embodiment of the disclosure, the blip is a statistically distinguishable signal in the drain current.

In one embodiment of the disclosure, the interacting agent includes an agent causing spore germination, an agent causing oxidative stress, an agent causing chemical damage or enzymatic destruction, an agent causing nutritional deficiency, UV irradiation, bacteriophages, an antibiotics, an agent causing essential ions deficiency, enzymes, radiation, or heat.

In one embodiment of the disclosure, two of the culturing chambers includes identical interacting agent and with different dosages or intensities.

In one embodiment of the disclosure, two of the culturing chambers includes different interacting agents.

In one embodiment of the disclosure, the pathogen-related information includes the existence of the pathogen, susceptibility of the pathogen to the interacting agent, dosage of the interacting agent sufficient to induce resistance of the pathogen, dosage of the interacting agent sufficient to suppress activity of the pathogen, and a fingerprint characteristic of the pathogen.

In one embodiment of the disclosure, the fingerprint characteristic of the pathogen includes the pathogen being alive or dead, being active or dormant, being contagious or noncommunicable, or being in one of phases comprising dormant, germination, outgrowth, vegetative, lag, stationary, or death.

In one embodiment of the disclosure, the biological sample includes body fluid, blood, or combinations thereof.

In one embodiment of the disclosure, at least a blip exists in the current, further including performing a monotonic increasing interacting agent dosage spread test or applying a different interacting agent to determine whether the pathogen in biological sample being resistant or sensitive to one of the interacting agents.

In one embodiment of the disclosure, no blip exists in the current, further including performing a monotonic decreasing interacting agent dosage spread test or applying a different interacting agent to determine whether the biological sample being free of pathogen or pathogen being sensitive to one of the interacting agents.

In one embodiment of the disclosure, the monotonic increasing interacting agent dosage spread test or the monotonic decreasing interacting agent dosage spread test each comprises a dosage of minimum inhibitory concentration (MIC) of the one of the interacting agents.

In one aspect, the present disclosure also provide a method for pathogen detection, including applying a biological sample to a pathogen detection chip, wherein the chip includes a culturing chamber configured to accommodate the biological sample, a sensor electrically coupled to the culturing chamber, and a reader configured to obtain an electrical signal from the sensor; driving the sensor; measuring the electrical signal from the sensor through the reader; and obtaining pathogen-related information of the biological sample based on the electrical signal.

In one embodiment of the disclosure, applying the biological sample to the pathogen detection chip includes contacting the biological sample to a solid surface of the sensor electrically coupled to the culturing chamber.

In one embodiment of the disclosure, the pathogen detection chip further comprises a microfluidic structure configured to inoculate, concentrate, dilute, or filter the biological sample to or in the culturing chamber.

### BRIEF DESCRIPTION OF THE DRAWINGS

FIG. 1 shows a cross section of a pathogen detection chip, in accordance with some embodiments of the present disclosure.
FIG. 2 depicts a schematic diagram of a sensor, in accordance with some embodiments of the present disclosure.
FIG. 3 shows a cross section of a pathogen detection chip, in accordance with some embodiments of the present disclosure.
FIG. 4A shows a value of current variation with respect to time for a biological sample free of any interacting agent, in accordance with some embodiments of the present disclosure.
FIG. 4B shows a number of colony-forming unit (CFU) with respect to time corresponding to the biological sample of FIG. 4A, in accordance with some embodiments of the present disclosure.
FIG. 4C shows pH values with respect to time corresponding to the biological sample of FIG. 5A, in accordance with some embodiments of the present disclosure.
FIG. 4D shows a value of current variation with respect to time for a biological sample resistant to an interacting agent, in accordance with some embodiments of the present disclosure.
FIG. 4E shows a number of colony-forming unit (CFU) with respect to time corresponding to the biological sample of FIG. 4D, in accordance with some embodiments of the present disclosure.
FIG. 4F shows pH values with respect to time corresponding to the biological sample of FIG. 4D, in accordance with some embodiments of the present disclosure.
FIG. 4G shows a value of current variation with respect to time for a biological sample sensitive to an interacting agent, in accordance with some embodiments of the present disclosure.
FIG. 4H shows a number of colony-forming unit (CFU) with respect to time corresponding to the biological sample of FIG. 4G, in accordance with some embodiments of the present disclosure.
FIG. 4I shows pH values with respect to time corresponding to the biological sample of FIG. 4G, in accordance with some embodiments of the present disclosure.
FIG. 5A shows a value of current variation with respect to time for a biological sample free of any interacting agent, in accordance with some embodiments of the present disclosure.
FIG. 5B shows a number of colony-forming unit (CFU) with respect to time corresponding to the biological sample of FIG. 5A, in accordance with some embodiments of the present disclosure.
FIG. 5C shows pH values with respect to time corresponding to the biological sample of FIG. 5A, in accordance with some embodiments of the present disclosure.
FIG. 5D shows a value of current variation with respect to time for a biological sample resistant to an interacting agent, in accordance with some embodiments of the present disclosure.
FIG. 5E shows a number of colony-forming unit (CFU) with respect to time corresponding to the biological sample of FIG. 5D, in accordance with some embodiments of the present disclosure.
FIG. 5F shows pH values with respect to time corresponding to the biological sample of FIG. 5D, in accordance with some embodiments of the present disclosure.
FIG. 5G shows a value of current variation with respect to time for a biological sample sensitive to an interacting agent, in accordance with some embodiments of the present disclosure.
FIG. 5H shows a number of colony-forming unit (CFU) with respect to time corresponding to the biological sample of FIG. 5G, in accordance with some embodiments of the present disclosure.
FIG. 5I shows pH values with respect to time corresponding to the biological sample of FIG. 5G, in accordance with some embodiments of the present disclosure.
FIG. 6A shows a value of current variation with respect to time for a biological sample free of any interacting agent, in accordance with some embodiments of the present disclosure.
FIG. 6B shows a number of colony-forming unit (CFU) with respect to time corresponding to the biological sample of FIG. 6A, in accordance with some embodiments of the present disclosure.
FIG. 6C shows pH values with respect to time corresponding to the biological sample of FIG. 6A, in accordance with some embodiments of the present disclosure.
FIG. 6D shows a value of current variation with respect to time for a biological sample resistant to an interacting agent, in accordance with some embodiments of the present disclosure.
FIG. 6E shows a number of colony-forming unit (CFU) with respect to time corresponding to the biological sample of FIG. 6D, in accordance with some embodiments of the present disclosure.
FIG. 6F shows pH values with respect to time corresponding to the biological sample of FIG. 6D, in accordance with some embodiments of the present disclosure.
FIG. 6G shows a value of current variation with respect to time for a biological sample sensitive to an interacting agent, in accordance with some embodiments of the present disclosure.
FIG. 6H shows a number of colony-forming unit (CFU) with respect to time corresponding to the biological sample of FIG. 6G, in accordance with some embodiments of the present disclosure.
FIG. 6I shows pH values with respect to time corresponding to the biological sample of FIG. 6G, in accordance with some embodiments of the present disclosure.
FIG. 7A shows a value of current variation with respect to time for a biological sample free of any interacting agent, in accordance with some embodiments of the present disclosure.
FIG. 7B shows a number of colony-forming unit (CFU) with respect to time corresponding to the biological sample of FIG. 7A, in accordance with some embodiments of the present disclosure.
FIG. 7C shows a value of current variation with respect to time for a biological sample resistant to an interacting agent, in accordance with some embodiments of the present disclosure.
FIG. 7D shows a number of colony-forming unit (CFU) with respect to time corresponding to the biological sample of FIG. 7C, in accordance with some embodiments of the present disclosure.
FIG. 7E shows a value of current variation with respect to time for a biological sample sensitive to an interacting agent, in accordance with some embodiments of the present disclosure.
FIG. 7F shows a number of colony-forming unit (CFU) with respect to time corresponding to the biological sample of FIG. 7E, in accordance with some embodiments of the present disclosure.
FIG. 8 shows a Table summarizing the electrical signal measured in FIG. 4A to FIG. 7E, in accordance with some embodiments of the present disclosure.
FIG. 9A and FIG. 9B show electrical signal with respect to time under different dosages of interacting agents, in accordance with some embodiments of the present disclosure.
FIG. 10A and FIG. 10B show electrical signal with respect to time under different dosages of interacting agents, in accordance with some embodiments of the present disclosure.
FIG. 11 illustrates a plurality of culturing wells or culturing chambers of a pathogen detection chip, each of the three samples are introduced into two culturing wells with differential interacting agent concentration spread, in accordance with some embodiments of the present disclosure.
FIG. 12 shows a flow chart of a method for pathogen detection, in accordance with some embodiments of the present disclosure.
FIG. 13 illustrates a plurality of culturing wells of a pathogen detection chip, each of the two samples are introduced into six culturing wells with monotonic increasing interacting agent concentration spread, in accordance with some embodiments of the present disclosure.
FIG. 14 illustrates a plurality of culturing wells of a pathogen detection chip, each of the two samples are introduced into six culturing wells with monotonic decreasing interacting agent concentration spread, in accordance with some embodiments of the present disclosure.

### DETAILED DESCRIPTION OF THE INVENTION

The claimed invention is defined in claims 1-12.

Unless otherwise defined herein, scientific and technical terms used in connection with the present invention shall have the meaning commonly understood by those of ordinary skill in the art. The meaning and scope of the terms should be clear; however, in the event of any latent ambiguity, definitions provided herein take precedence over any dictionary or extrinsic definition.

Unless otherwise required by context, singular terms shall include the plural and plural terms shall include the singular. For example, the term "a" or "an," as used herein, is defined as one or more than one.

In the description that follows, a number of terms are used and the following definitions are provided to facilitate understanding of the claimed subject matter. Terms that are not expressly defined herein are used in accordance with their plain and ordinary meanings.

As used herein, the term "or" in the claims is used to mean "and/or" unless explicitly indicated to refer to alternatives only or the alternatives are mutually exclusive.

As used herein, the term "a pathogen" refers to an organism that causes an infection or infectious disease. Preferably, the pathogen is single-celled organism. More preferably, the cell is a microorganism. Examples of the microorganism include but are not limited to archaea, virus, bacteria and eukaryotes such as protists, fungi and plants. Preferably, the pathogen according to the disclosure is culturable. In another aspect, the pathogen according to the disclosure is isolated, non-isolated or partially isolated.

As used herein, the term "a pathogen detection" refers to a process for detecting a specific characteristic for distinguishing a pathogen species from others. It is believed, though not intended to be restricted by any theoretical, each pathogen species has specific growth pattern and the corresponding metabolites may produce detectable and specific electrical signal, and by collecting the electrical signal during a course of culturing, a fingerprint pattern identifying the pathogen can be obtained.

As used herein, the term "a biological sample" refers to a sample containing the pathogen. The biological sample according to the disclosure is derived from a naturally occurring origin or derived from artificial manipulation. Preferably, the biological sample is derived from a naturally occurring origin such as a subject or patient, an extract, bodily fluid, tissue biopsy, liquid biopsy, or cell culture. Preferably, the biological sample is derived from artificial manipulation such as culture medium or food composition. In another aspect, the biological sample is processed according to the reaction of detection. For example, the pH value or ion strength of the biological sample may be adjusted. In one preferred embodiment of the disclosure, the biological sample is body fluid, blood, or combinations thereof.

As used herein, the terms "a subject" and "a patient" are used interchangeably herein and will be understood to refer to a warm blood animal, particularly a mammal. Non-limiting examples of animals within the scope and meaning of this term include guinea pigs, dogs, cats, rats, mice, horses, goats, cattle, sheep, zoo animals, non-human primates, and humans. Preferably, the subject is suspected as an infection patient. More preferably, the subject is a suspected sepsis patient.

As used herein, the term "an interacting agent" refers to an agent that interacts with a pathogen or causes stress to growth of a pathogen. As used herein, the term "growth of a pathogen" refers to the difference of a pathogen culture before and after a period of culturing. Examples of the difference include but are not limited to cell counts, cell growth rates, metabolism, phases, or stages. Examples of the growth include but are not limited to being alive or dead, being active or dormant, being contagious or noncommunicable, growth phase changes, dormant growth, germination, outgrowth, or vegetative growth. The method according to the disclosure is not only able to detect the pathogen counts or growth rates, but also to detect several statuses of the pathogen growth. Examples of the interacting agent include but are not limited to an agent causing spore germination, an agent causing oxidative stress, an agent causing chemical damage or enzymatic destruction, an agent causing nutritional deficiency, UV irradiation, bacteriophages, an antibiotics, an agent causing essential ions deficiency, enzymes, radiation, or heat. Some bacteria may form spores to produce a dormant and highly resistant cell to preserve the cell's genetic material in times of extreme stress, such as environmental assaults; and the term "spore germination" means the event that result in the loss of the spore-specific properties. Examples of the agent causing oxidative stress include but are not limited to excessive irons or free radicals. Examples of the interacting agents causing nutritional deficiency include but are not limited to an agent causing phosphates deficiency or an agent causing amino acids deficiency. Examples of the agents causing essential ion deficiency include but are not limited to Ethylenediaminetetraacetic acid (EDTA) to remove Ca, Mg, Fe, Co, Ni, Na, or Mn in the biological sample. Examples of the interacting agents being antibiotic include but are not limited to amoxicillin, gentamicin, chloramphenicol, ampicillin, kanamycin, tetracycline, spectinomycin, doxycycline, cephalexin, ciprofloxacin, clindamycin, metronidazole, azithromycin, sulfamethoxazole, trimethoprim, clavulanate, or levofloxacin.

Examples of the "pathogen-related information" include but are not limited to the existence of the pathogen, susceptibility or sensitivity of the pathogen to the interacting agent, dosage of the interacting agent sufficient to induce resistance of the pathogen, dosage of the interacting agent sufficient to suppress activity of the pathogen, or a fingerprint characteristic of the pathogen. Examples of the fingerprint characteristic of the pathogen include but are not limited to the pathogen being alive or dead, being active or dormant, being contagious or noncommunicable, being in one of phases comprising dormant, germination, outgrowth, vegetative, lag, stationary, or death.

As used herein, the term "susceptibility of a pathogen to an interacting agent" refers to sensitivity of the pathogen to the agent. In other words, the method according to the disclosure is provided to screen if the interacting agent affects the growth of the pathogen, such as suppress or inhibit the growth of the pathogen.

In order to facilitate determining the electrical signals, the sample preferably contacts with a solid surface. As used herein, the term "solid surface" refers to a solid support including but not limited to a silicon, silicon oxide, polymer, paper, fabric, or glass. Preferably, the solid surface is an electrical sensor or an electromagnetic sensor. Preferably, the solid surface to be employed varies depending on an electrical change detecting element as mentioned below. For example, when the method adopts a field-effect transistor to detect the electrical change, the solid surface is a transistor surface of the field-effect transistor; when the method adopts a surface plasmon resonance, the solid surface is a metal surface of a surface plasmon resonance.

Preferably, the solid surface is coupled with an electrical signal detecting element for detecting the change of electrical signal. In some embodiments, the electrical signal detecting element is a field-effect transistor or a surface plasmon resonance device, a CMOS image sensor, a backside illumination sensor, or an organic or non-organic miniaturized electrode. Preferably, the field-effect transistor is a nanowire field-effect transistor or a nano-plate field-effect transistor.

In a preferred embodiment of the invention, the material of the solid surface is silicon, preferably polycrystalline silicon or single crystalline silicon; more preferably polycrystalline silicon. Polycrystalline silicon is cheaper than single crystalline silicon, but the grain boundary in the polycrystalline silicon may hinder the electron transfer mobility. Such phenomenon makes the solid surface uneven and quantification difficult. Furthermore, ions may penetrate into the grain boundary of the polycrystalline silicon and cause detection failure in solution. In addition, polycrystalline silicon is not stable in air. The abovementioned drawbacks, however, would not interfere with the function of the method according to the present disclosure.

As used herein, the term "change of electrical signal" refers to the variation of an electrical signal of a cell culture during the course of culturing. The change of electrical signal according to the disclosure includes but is not limited to variation of electrical current or differential electrical current. The change of electrical signal can be measured using suitable electronic device or system. The change of electrical signal includes but is not limited to a change of electrical charge, a change of electrical current, a change of electrical resistance, a change of threshold voltage, a change of electrical conductivity, a change of electric field, a change of electrical capacitance. Preferably, the change of electrical signal is the change of a threshold voltage of a field effect transistor and hence a differential drain current can be measured.

It is believed, though not intended to be restricted by any theoretical, that since the sensitivity of detecting the pathogen using the method for pathogen detection described herein can be as low as less than 100 colony-forming unit (CFU) per 100 µL, and taking only few hours of culturing (e.g., less than 12 hours). The susceptibility of the interacting agent to the growth of the pathogen can be identified rapidly with relatively low concentration of pathogen.

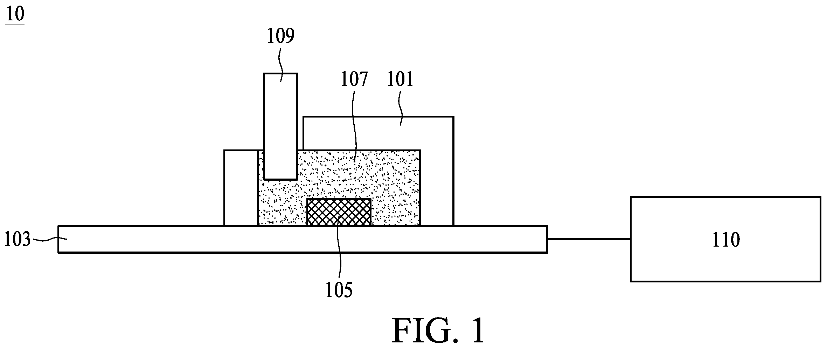

Referring to FIG. 1, FIG. 1 shows a cross section of a pathogen detection chip 10, in accordance with some embodiments of the present disclosure. As shown in FIG. 1, the pathogen detection chip 10 includes a culturing chamber 101 disposed on a carrier 103, for example, a circuit board or any substrate compatible to semiconductor manufacture. The culturing chamber 101 is configured to accommodate a culturing medium 107 and a sensing chip 105 immersed or partially immersed in the culturing medium 107. As previously described, the culturing medium 107 is in contact with a solid surface of the sensing chip 105 to generate electrical signals. In some embodiments, the solid surface can refer to a source, a drain, a channel, or a passivation layer of a field effect transistor (FET) or a Bio-FET. The sensing chip 105 is devised to be in direct contact with the culturing medium 107 which is at least composed of biological sample and/or interacting agents. A reference electrode 109 is integrated in a way that the reference electrode 109 may be in contact with the culturing medium 107 so as to apply a desired bias to the culturing medium 107. In some embodiments, the reference electrode 109 can be composed of metal such as Ag or AgCl. The reader 110 electrically connected to the sensing chip 105 is configured to read the electrical signal generated by the sensing chip 105 as a result of the interaction between the solid surface of the FET and the culturing medium 107 for a predetermined time period. In some embodiments, the reader 110 is electrically connected to the sensing chip 105 via the conductive connections in the carrier 103 (e.g., the conductive traces in a printed circuit board or the redistribution layer in a substrate suitable for semiconductor manufacturing).

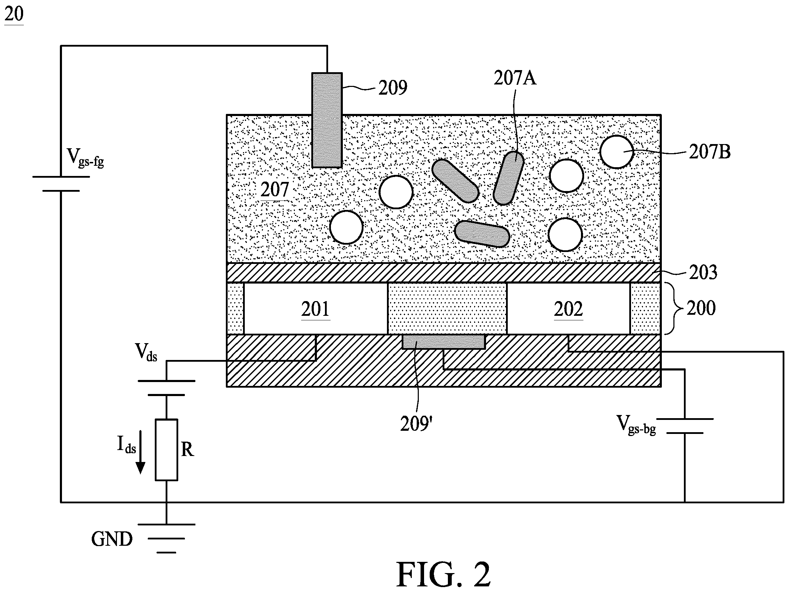

Referring to FIG. 2, FIG. 2 depicts a schematic diagram of a sensor 20, in accordance with some embodiments of the present disclosure. As shown in FIG. 2, the sensor 20 includes a transistor, a field effect transistor (FET), or a bio-FET having conductive regions such as a source 201 and a drain 202 in a semiconductor layer 200. A channel region may be disposed between the source 201 and the drain 202. A first gate 209 or a front gate described herein is in contact with the culturing medium 207, a second gate 209' or a back gate described herein free from being in contact with the culturing medium 207. As exemplified in FIG. 2, the culturing medium 207 includes biological sample 207A and interacting agent 207B that imposing survival stress or vital stress to the biological sample 207A. The biological sample 207A may be in a form of body fluid, blood, sweat, urine, or combinations thereof. The biological sample 207A may include at least one type of bacteria. In some embodiments, the interacting agent 207B may include antibiotics, enzymes, irons, free radicals, phosphate, amino acids, and chelating agents such as Ethylenediaminetetraacetic acid (EDTA). In some embodiments, the interacting agent 207B may not be chemical substance but a form of energy such as radiation or heat. Optionally, a passivation layer 203 can be disposed to cover a portion of the surface of the semiconductor layer 200 and exposing another portion of the semiconductor layer 200 to be in contact with the culturing medium 207.

As illustrated in FIG. 2, a front gate voltage V_{gs-fg} and a back gate voltage V_{gs-bg} can be established. The drain voltage V_{ds} may cause a drain current I_{ds} to be measured as the electrical signal. In some embodiments, during the course of culturing the biological sample 207A, metabolites and/or the ions of the biological sample 207A generated due to the interaction of which with the interacting agents 207B may cause the front gate voltage V_{gs-fg} to change and thus the change of drain voltage V_{ds} as well as the measured drain current I_{ds}. By analyzing the electrical signal of the differential drain current ΔI_{ds}, several characteristics of the biological sample 207A can be determined within a short time frame and at a relative low sample concentration. In some embodiments, the sensor 20 may occupies a footprint of 5µm by 5µm. A culturing medium 207 of about 200µL can be loaded to the culturing chamber (shown in FIG. 1). A bias of +1.8V can be applied to the first gate 209 and a bias of -0.5V can be applied to the second gate 209' during the measurement of drain current I_{ds}.

Two approaches can be used to measure the differential drain current ΔI_{ds}. One approach is to subtract the drain current I_{ds} measured from a control group of the sensor 20 where no biological sample is introduced into the culturing medium 207 from the drain current I_{ds} measured from an experimental group of the sensor 20 where biological sample is introduced into the culturing medium 207 during the course of culturing. The other approach is to subtract the initial drain current I_{ds} (e.g. at t=0) from the drain current I_{ds} measured from an experimental group of the sensor 20 where biological sample is introduced into the culturing medium 207 during the course of culturing. The latter approach can be adopted for real-time pathogen detection, and the former approach can be adopted in a more systematic study where the concentration of biological sample and the pH value of the culturing medium 207 can be recorded simultaneously.

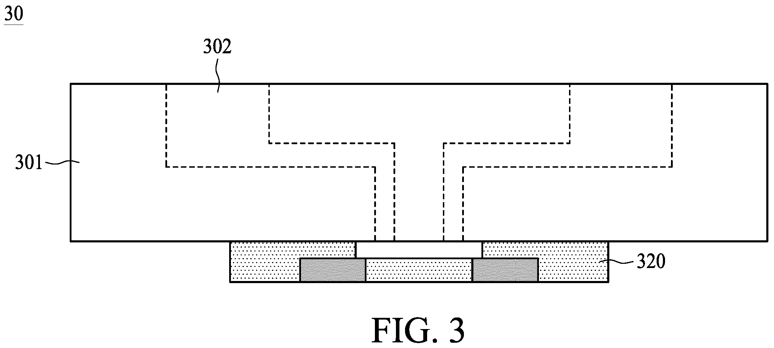

Referring to FIG. 3, FIG. 3 shows a cross section of a pathogen detection chip 30, in accordance with some embodiments of the present disclosure. The pathogen detection chip 30 includes an incubation unit 301 affixed to the sensor 320 which can be substantially identical to the sensor 20 described in FIG. 2. The incubation unit 301 may include a microfluidic structure 302 configured to inoculate concentrate, dilute, or filter the biological sample to or in the corresponding culturing chamber. In some embodiments, the pathogen detection chip 30 may include a plurality of culturing chambers each with an independent sensor devised therein. Users of the pathogen detection chip 30 may inoculate the biological sample to the microfluidic structure 302 so that the biological sample can be evenly distributed to the plurality of culturing chambers. Prior to the biological sample inoculation, various interacting agents with identical or different concentration can be loaded to each of the plurality of culturing chamber, depending on the characteristic (e.g., existence of the pathogen, susceptibility of the pathogen to the interacting agent, dosage of the interacting agent sufficient to induce drug resistance of the pathogen, and a fingerprint characteristic of the pathogen) of the biological sample to be screened.

The present disclosure provides a method for pathogen detection utilizing the pathogen detection chip 10 and 30 described in FIG. 1 and FIG. 3, as well as the sensor 20 described in FIG. 2 of the present disclosure. Referring to FIG. 1, FIG. 2, and FIG. 3, the method includes (1) applying a biological sample 207A to a culturing chamber 101 comprising an interacting agent 207B; (2) driving a sensor 20 electrically coupled to the biological sample 207A in the culturing chamber 101; (3) measuring an electrical signal (e.g., the drain current I_{ds}) from the sensor 20; and (4) obtaining pathogen-related information of the biological sample 207A based on the electrical signal. As will be addressed in the following description, the electrical signal observed in some of the embodiments can be the differential drain current ΔI_{ds}. By analyzing the electrical signal pattern during the course of detection, different characteristics of the biological sample 207A can be determined in a short period of time (e.g., less than 12 hours) and at a low initial biological sample concentration (e.g., less than 100 CFU per 100 µL).

### Example 1A

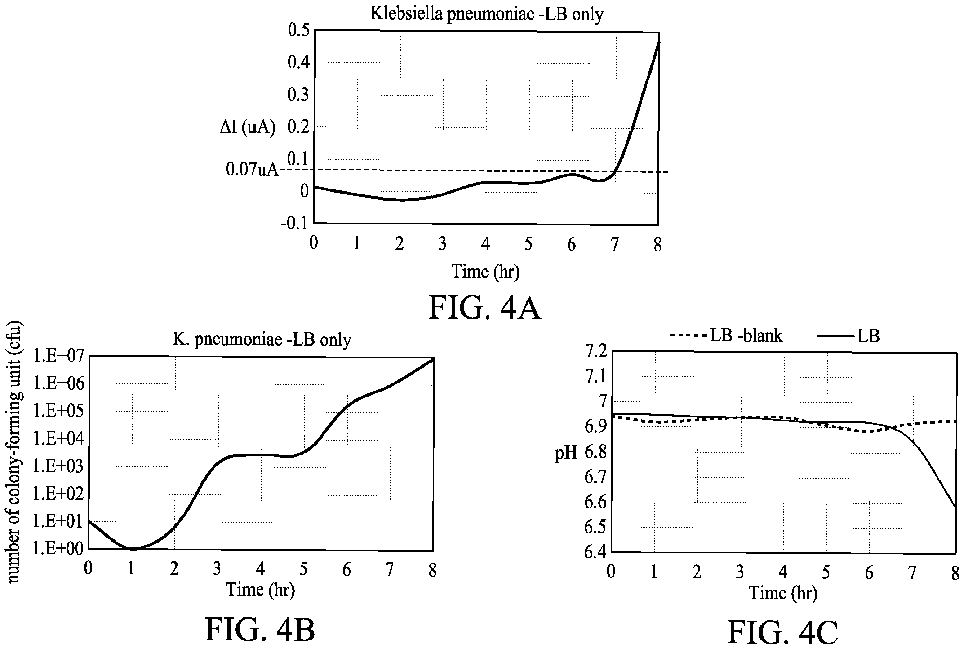

Referring to FIG. 4A, FIG. 4B, and FIG. 4C, FIG. 4A shows a value of current variation (e.g., differential drain current ΔI_{ds}, or equivalent to ΔI_{ds} previously described) with respect to time (Hr) for a biological sample being resistant to a interacting agent, FIG. 4B shows a number of colony-forming unit (CFU) with respect to time (Hr) corresponding to the biological sample of FIG. 4A, and FIG. 4C shows pH values of the culturing medium with respect to time corresponding to the biological sample of FIG. 4A, in accordance with some embodiments of the present disclosure. Klebsiella pneumoniae is used as biological sample and no interacting agent is applied in Example 1A.

To prepare the culturing medium, 200 µL of non-buffered LB broth having predetermined dosages (including zero dosage) of interacting agent was inoculated with predetermined concentration of Klebsiella pneumoniae and cultured at a temperature of 37°C. The sample was subjected to the pathogen detection using the pathogen detection chip and sensor described herein with a front gate voltage of +1.8V and a back gate voltage of -0.5V for drain current I_{ds} measurement at predetermined time points (e.g., at each hour). The differential drain current ΔI_{ds} can be determined using the two approaches previously described, including (1) subtracting the drain current I_{ds} measured from a control group from the drain current I_{ds} measured from an experimental group during the course of culturing; and (2) subtracting the initial drain current I_{ds} (e.g. at t=0) from the drain current I_{ds} measured from an experimental group during the course of culturing. When the former approach is taken, the concentration of biological sample can be determined by agar plating under the same environmental condition.

As shown in FIG. 4A, since the differential drain current ΔI_{ds} (or ΔI described herein) does not appear to have a statistically distinguishable signal therein, or in some simplified and specific examples for illustration only, there is no local maximum value greater than or equal to 3 times of the base value during the first 7 hours of culturing, no blip in the differential drain current ΔI_{ds} can be identified. As shown in FIG. 4B, the number of colony-forming unit (CFU) obtained from agar plating shows a trend of monotonic increasing after the 1^{st} hour, and as shown in FIG. 4C, the pH value of the culturing medium approximately remains unchanged or with a variation less than 0.3 with respect to the initial pH value (t=0), indicating that the electrical signal pattern shown in FIG. 4A corresponds to the biological sample (e.g., Klebsiella pneumoniae) being in an environment free of interacting agent.

### Example 1B

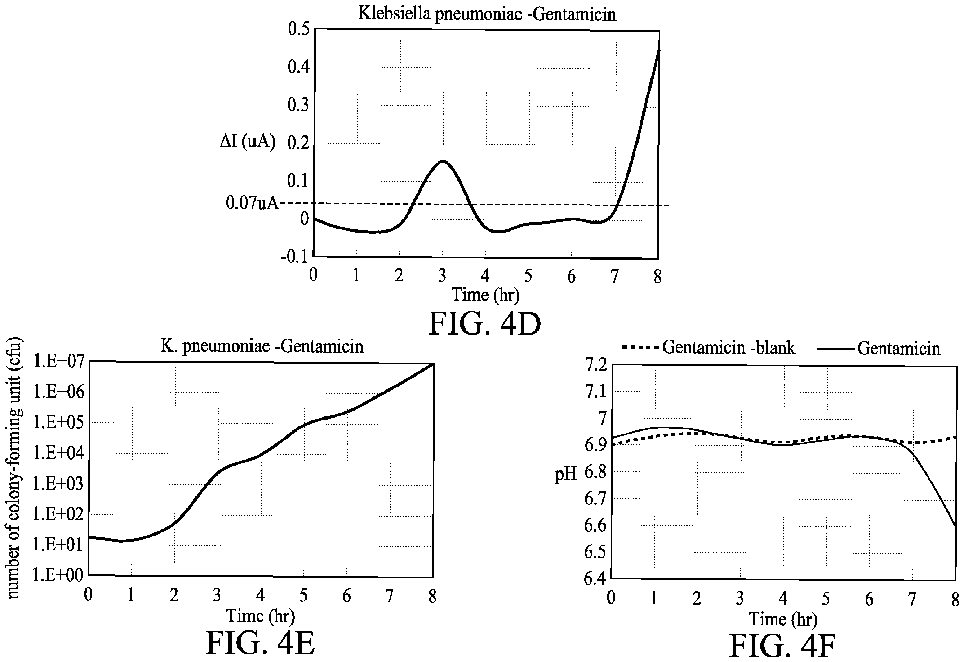

Referring to FIG. 4D, FIG. 4E, and FIG. 4F, FIG. 4D shows a value of current variation (e.g., differential drain current ΔI_{ds}, or equivalent to ΔI_{ds} previously described) with respect to time (Hr) for a biological sample being resistant to a interacting agent, FIG. 4E shows a number of colony-forming unit (CFU) with respect to time (Hr) corresponding to the biological sample of FIG. 4D, and FIG. 4F shows pH values of the culturing medium with respect to time corresponding to the biological sample of FIG. 4D, in accordance with some embodiments of the present disclosure. Klebsiella pneumoniae is used as biological sample and Gentamicin is used as an interacting agent in Example 1B.

Sample preparation and measurement condition can be referred to those in Example 1A and are not repeated here for brevity. As shown in FIG. 4D, a blip of about 0.15µA can be identified at the 3^{rd} hour of the culturing. When a local maximum ΔI_{ds} value is greater than or equal to about 3 times of a base ΔI_{ds} value (e.g., the corresponding ΔI_{ds} value measured in the control group without biological sample or the corresponding ΔI_{ds} value measured in the experimental group with biological sample at t=0), a blip can be identified in the differential drain current ΔI_{ds} measurement. In some embodiments, since the sensor possesses finite signal variation associated with the stability of signal processing system, a differential drain current ΔI_{ds} of about ±0.01µA to about ±0.03µA can be observed as background noise. People having ordinary skill in the art can appreciate that sensors with different signal processing system may provide different degree of electrical signal stability and hence the aforesaid background noise may change accordingly. Determining whether the electrical signal constitutes a blip should take the device-sensitive factor such as background noise into consideration. For example, in Example 1B, a differential drain current ΔI_{ds} greater than 0.07µA can be considered as a blip in the electrical signal, a differential drain current ΔI_{ds} greater than 0.03µA but lower than 0.07µA can be considered as a sub-blip in the electrical signal, and a differential drain current ΔI_{ds} lower than 0.03µA does not constitute a blip or a sub-blip. As shown in FIG. 4E, the number of colony-forming unit (CFU) obtained from agar plating shows a trend of monotonic increasing after the 1^{st} hour, and as shown in FIG. 4F, the pH value of the culturing medium approximately remains unchanged or with a variation less than 0.3 with respect to the initial pH value (t=0), indicating that the electrical signal pattern shown in FIG. 4D corresponds to the biological sample (e.g., Klebsiella pneumoniae) being resistant to the interacting agent (e.g., Gentamicin).

### Example 1C

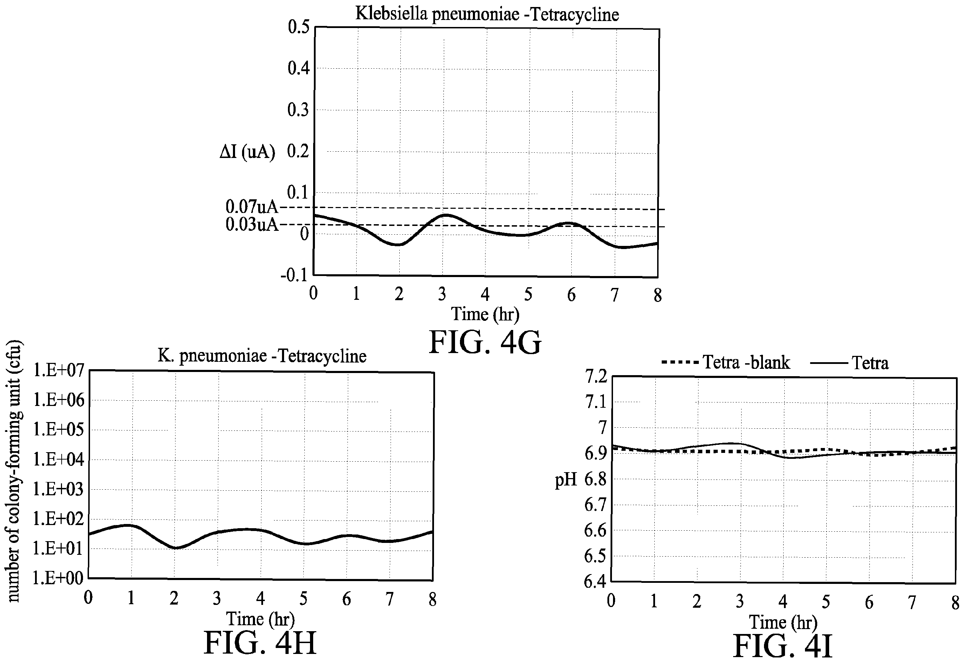

Referring to FIG. 4G, FIG. 4H, and FIG. 4I, FIG. 4G shows a value of current variation (e.g., differential drain current ΔI_{ds}, or equivalent to ΔI_{ds} previously described) with respect to time (Hr) for a biological sample being sensitive to a interacting agent, FIG. 4H shows a number of colony-forming unit (CFU) with respect to time (Hr) corresponding to the biological sample of FIG. 4G, and FIG. 4I shows pH values of the culturing medium with respect to time corresponding to the biological sample of FIG. 4G, in accordance with some embodiments of the present disclosure. Klebsiella pneumoniae is used as biological sample and Tetracycline is used as an interacting agent in Example 1C.

Sample preparation and measurement condition can be referred to those in Example 1A and are not repeated here for brevity. As shown in FIG. 4G, the differential drain current ΔI_{ds} does not appear to have a local maximum value greater than or equal to 3 times of the base value, but demonstrating two local maxima lower than 3 times of the base value at the 3^{rd} hour and the 6^{th} hour, respectively. For example, in Example 1C, the differential drain current ΔI_{ds} are lower than 0.07µA but greater than 0.03µA at the 3^{rd} hour and the 6^{th} hour, therefore two sub-blips can be identified in the electrical signal. As shown in FIG. 4H, the number of colony-forming unit (CFU) obtained from agar plating shows a trend of neither increasing or decreasing, and as shown in FIG. 4I, the pH value of the culturing medium approximately remains unchanged or with a variation less than 0.3 with respect to the initial pH value (t=0), indicating that the electrical signal pattern shown in FIG. 4G corresponds to the biological sample (e.g., Klebsiella pneumoniae) being sensitive to the interacting agent (e.g., Tetracycline).

### Example 2A

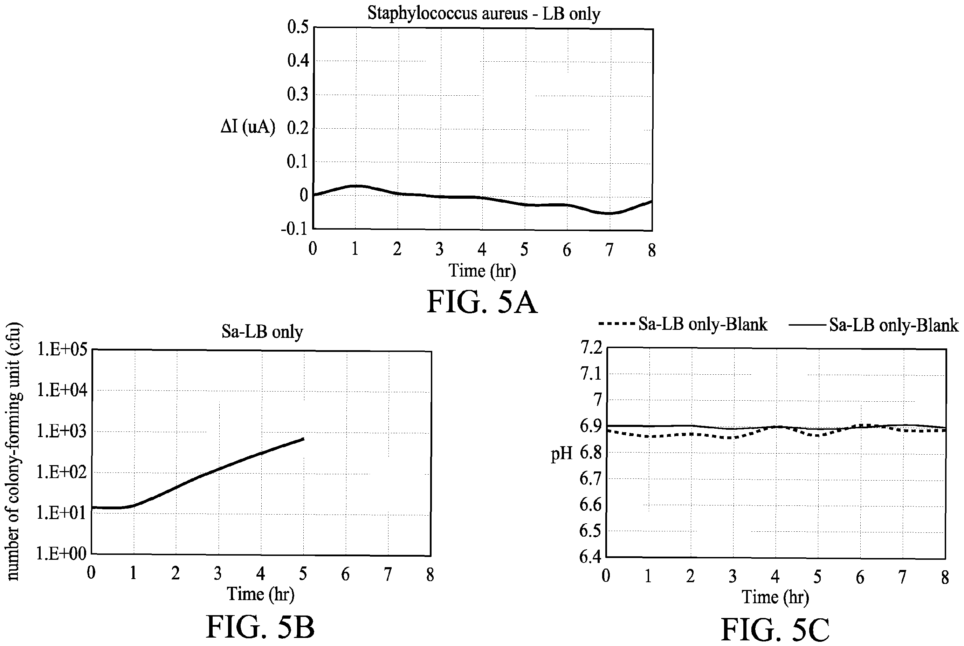

Referring to FIG. 5A, FIG. 5B, and FIG. 5C, FIG. 5A shows a value of current variation (e.g., differential drain current ΔI_{ds}, or equivalent to ΔI_{ds} previously described) with respect to time (Hr) for a biological sample being resistant to a interacting agent, FIG. 5B shows a number of colony-forming unit (CFU) with respect to time (Hr) corresponding to the biological sample of FIG. 5A, and FIG. 5C shows pH values of the culturing medium with respect to time corresponding to the biological sample of FIG. 5A, in accordance with some embodiments of the present disclosure. Staphylococcus aureus is used as biological sample and no interacting agent is applied in Example 2A.

To prepare the culturing medium, 200 µL of non-buffered LB broth having predetermined dosages (including zero dosage) of interacting agent was inoculated with predetermined concentration of Staphylococcus aureus and cultured at a temperature of 37°C. The sample was subjected to the pathogen detection using the pathogen detection chip and sensor described herein with a front gate voltage of +1.8V and a back gate voltage of -0.5V for drain current I_{ds} measurement at predetermined time points (e.g., at each hour). The differential drain current ΔI_{ds} can be determined using the two approaches previously described, including (1) subtracting the drain current I_{ds} measured from a control group from the drain current I_{ds} measured from an experimental group during the course of culturing; and (2) subtracting the initial drain current I_{ds} (e.g. at t=0) from the drain current I_{ds} measured from an experimental group during the course of culturing. When the former approach is taken, the concentration of biological sample can be determined by agar plating under the same environmental condition.

As shown in FIG. 5A, since the differential drain current ΔI_{ds} does not appear to have a local maximum value greater than or equal to 3 times of the base value during the first 8 hours of culturing, no blip in the differential drain current ΔI_{ds} can be identified. As shown in FIG. 5B, the number of colony-forming unit (CFU) obtained from agar plating shows a trend of monotonic increasing, and as shown in FIG. 5C, the pH value of the culturing medium approximately remains unchanged or with a variation less than 0.3 with respect to the initial pH value (t=0), indicating that the electrical signal pattern shown in FIG. 5A corresponds to the biological sample (e.g., Staphylococcus aureus) being in an environment free of interacting agent.

### Example 2B

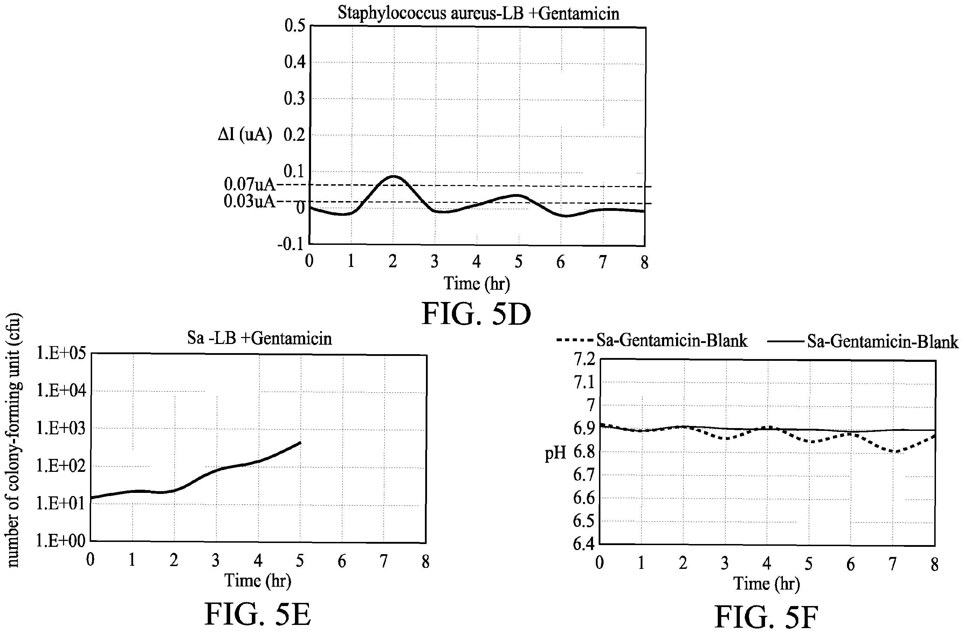

Referring to FIG. 5D, FIG. 5E, and FIG. 5F, FIG. 5D shows a value of current variation (e.g., differential drain current ΔI_{ds}, or equivalent to ΔI_{ds} previously described) with respect to time (Hr) for a biological sample being resistant to a interacting agent, FIG. 5E shows a number of colony-forming unit (CFU) with respect to time (Hr) corresponding to the biological sample of FIG. 5D, and FIG. 5F shows pH values of the culturing medium with respect to time corresponding to the biological sample of FIG. 5D, in accordance with some embodiments of the present disclosure. Staphylococcus aureus is used as biological sample and Gentamicin is used as an interacting agent in Example 2B.

Sample preparation and measurement condition can be referred to those in Example 2A and are not repeated here for brevity. As shown in FIG. 5D, two local maxima can be identified at the 2^{nd} hour and the 5^{th} hour, respectively. For example, in Example 2B, a differential drain current ΔI_{ds} greater than 0.07µA can be considered as a blip in the electrical signal, a differential drain current ΔI_{ds} greater than 0.03µA but lower than 0.07µA can be considered as a sub-blip in the electrical signal, and a differential drain current ΔI_{ds} lower than 0.03µA does not constitute a blip or a sub-blip. As a result, the differential drain current ΔI_{ds} of FIG. 5D shows a blip at the 2^{nd} hour and a sub-blip at the 5^{th} hour.

As shown in FIG. 5E, the number of colony-forming unit (CFU) obtained from agar plating shows a trend of monotonic increasing, and as shown in FIG. 5F, the pH value of the culturing medium approximately remains unchanged or with a variation less than 0.3 with respect to the initial pH value (t=0), indicating that the electrical signal pattern shown in FIG. 5D corresponds to the biological sample (e.g., Staphylococcus aureus) being resistant to the interacting agent (e.g., Gentamicin).

### Example 2C

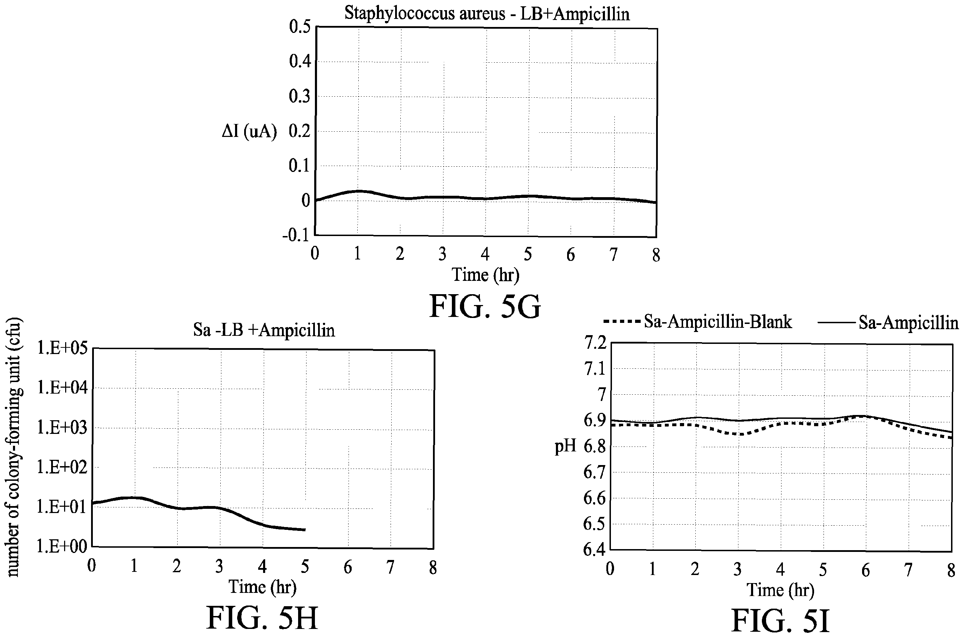

Referring to FIG. 5G, FIG. 5H, and FIG. 5I, FIG. 5G shows a value of current variation (e.g., differential drain current ΔI_{ds}, or equivalent to ΔI_{ds} previously described) with respect to time (Hr) for a biological sample being sensitive to a interacting agent, FIG. 5H shows a number of colony-forming unit (CFU) with respect to time (Hr) corresponding to the biological sample of FIG. 5G, and FIG. 5I shows pH values of the culturing medium with respect to time corresponding to the biological sample of FIG. 5G, in accordance with some embodiments of the present disclosure. Staphylococcus aureus is used as biological sample and Ampicillin is used as an interacting agent in Example 2C.

Sample preparation and measurement condition can be referred to those in Example 2A and are not repeated here for brevity. As shown in FIG. 5G, the differential drain current ΔI_{ds} does not appear to have a local maximum value greater than or equal to 3 times of the base value. For example, in Example 2C, the differential drain current ΔI_{ds} are all lower than 0.03µA throughout the first 8 hour of culturing, therefore no blips or sub-blips can be identified in the electrical signal. As shown in FIG. 5H, the number of colony-forming unit (CFU) obtained from agar plating shows a trend of monotonic decreasing after the 1^{st} hour, and as shown in FIG. 5I, the pH value of the culturing medium approximately remains unchanged or with a variation less than 0.3 with respect to the initial pH value (t=0), indicating that the electrical signal pattern shown in FIG. 5G corresponds to the biological sample (e.g., Staphylococcus aureus) being sensitive to the interacting agent (e.g., Ampicillin).

### Example 3A

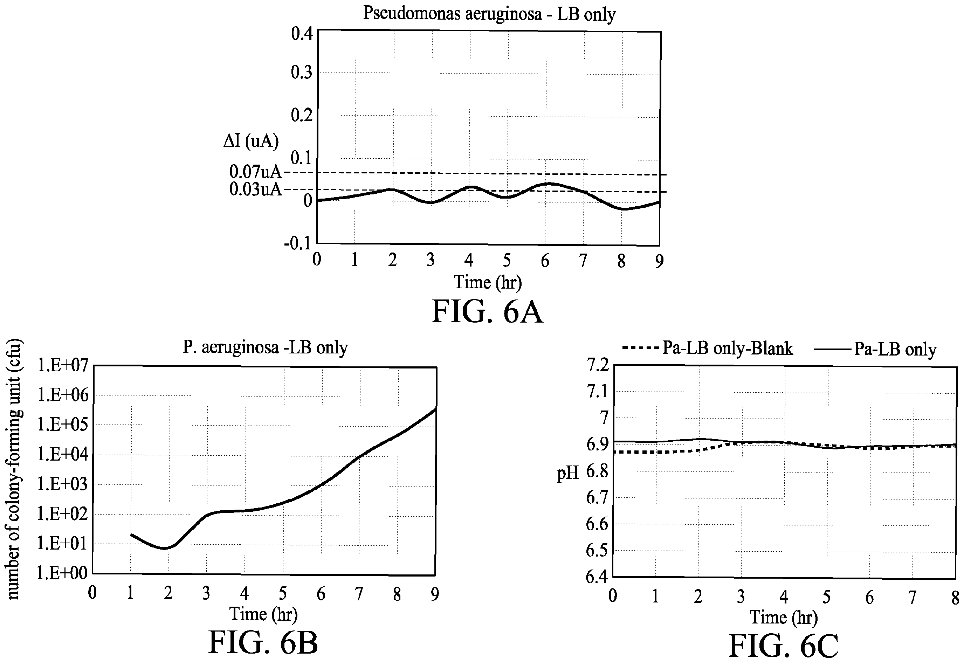

Referring to FIG. 6A, FIG. 6B, and FIG. 6C, FIG. 6A shows a value of current variation (e.g., differential drain current ΔI_{ds}, or equivalent to ΔI_{ds} previously described) with respect to time (Hr) for a biological sample being resistant to a interacting agent, FIG. 6B shows a number of colony-forming unit (CFU) with respect to time (Hr) corresponding to the biological sample of FIG. 6A, and FIG. 6C shows pH values of the culturing medium with respect to time corresponding to the biological sample of FIG. 6A, in accordance with some embodiments of the present disclosure. Pseudomonas aeruginosa is used as biological sample and no interacting agent is applied in Example 3A.

To prepare the culturing medium, 200 µL of non-buffered LB broth having predetermined dosages (including zero dosage) of interacting agent was inoculated with predetermined concentration of Pseudomonas aeruginosa and cultured at a temperature of 37°C. The sample was subjected to the pathogen detection using the pathogen detection chip and sensor described herein with a front gate voltage of +1.8V and a back gate voltage of -0.5V for drain current I_{ds} measurement at predetermined time points (e.g., at each hour). The differential drain current ΔI_{ds} can be determined using the two approaches previously described, including (1) subtracting the drain current I_{ds} measured from a control group from the drain current I_{ds} measured from an experimental group during the course of culturing; and (2) subtracting the initial drain current I_{ds} (e.g. at t=0) from the drain current I_{ds} measured from an experimental group during the course of culturing. When the former approach is taken, the concentration of biological sample can be determined by agar plating under the same environmental condition.

As shown in FIG. 6A, the differential drain current ΔI_{ds} does not appear to have a local maximum value greater than or equal to 3 times of the base value during the first 9 hours of culturing. However, the differential drain current ΔI_{ds} appear to have two local maxima lower than 0.07µA but greater than 0.03µA at the 4^{th} hour and the 6^{th} hour, zero blip and two sub-blips in the differential drain current ΔI_{ds} can be identified. As shown in FIG. 6B, the number of colony-forming unit (CFU) obtained from agar plating shows a trend of monotonic increasing after the 2^{nd} hour, and as shown in FIG. 6C, the pH value of the culturing medium approximately remains unchanged or with a variation less than 0.3 with respect to the initial pH value (t=0), indicating that the electrical signal pattern shown in FIG. 6A corresponds to the biological sample (e.g., pseudomonas aeruginosa) being in an environment free of interacting agent.

### Example 3B

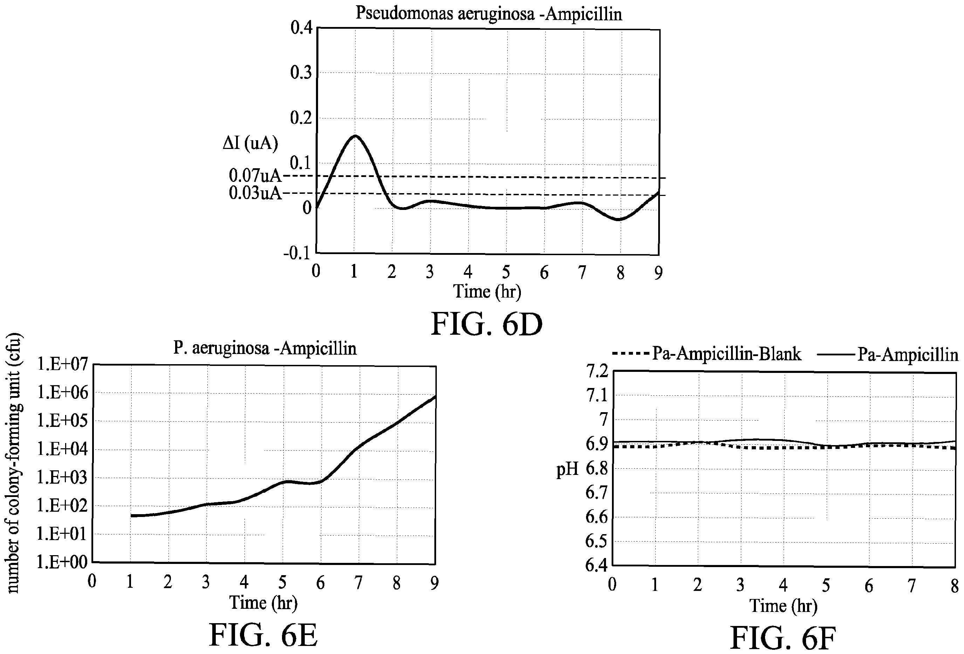

Referring to FIG. 6D, FIG. 6E, and FIG. 6F, FIG. 6D shows a value of current variation (e.g., differential drain current ΔI_{ds}, or equivalent to ΔI_{ds} previously described) with respect to time (Hr) for a biological sample being resistant to a interacting agent, FIG. 6E shows a number of colony-forming unit (CFU) with respect to time (Hr) corresponding to the biological sample of FIG. 6D, and FIG. 6F shows pH values of the culturing medium with respect to time corresponding to the biological sample of FIG. 6D, in accordance with some embodiments of the present disclosure. Pseudomonas aeruginosa is used as biological sample and Ampicillin is used as an interacting agent in Example 3B.

Sample preparation and measurement condition can be referred to those in Example 3A and are not repeated here for brevity. As shown in FIG. 6D, the differential drain current ΔI_{ds} appear to have a local maximum value greater than or equal to 3 times of the base value at the 1^{st} hour of culturing. For example, in Example 3B, a differential drain current ΔI_{ds} of about 0.16µA can be identified as a blip in the electrical signal.

As shown in FIG. 6E, the number of colony-forming unit (CFU) obtained from agar plating shows a trend of monotonic increasing, and as shown in FIG. 6F, the pH value of the culturing medium approximately remains unchanged or with a variation less than 0.3 with respect to the initial pH value (t=0), indicating that the electrical signal pattern shown in FIG. 6D corresponds to the biological sample (e.g., pseudomonas aeruginosa) being resistant to the interacting agent (e.g., Ampicillin).

### Example 3C

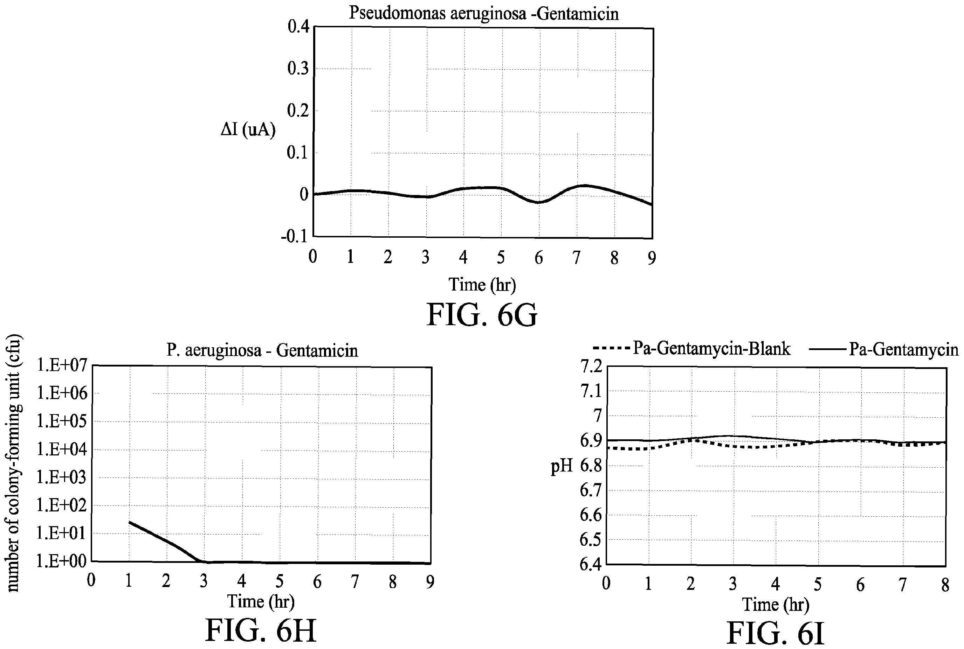

Referring to FIG. 6G, FIG. 6H, and FIG. 6I, FIG. 6G shows a value of current variation (e.g., differential drain current ΔI_{ds}, or equivalent to ΔI_{ds} previously described) with respect to time (Hr) for a biological sample being sensitive to a interacting agent, FIG. 6H shows a number of colony-forming unit (CFU) with respect to time (Hr) corresponding to the biological sample of FIG. 6G, and FIG. 6I shows pH values of the culturing medium with respect to time corresponding to the biological sample of FIG. 6G, in accordance with some embodiments of the present disclosure. Pseudomonas aeruginosa is used as biological sample and Gentamicin is used as an interacting agent in Example 3C.

Sample preparation and measurement condition can be referred to those in Example 3A and are not repeated here for brevity. As shown in FIG. 6G, the differential drain current ΔI_{ds} does not appear to have a local maximum value greater than or equal to 3 times of the base value. For example, in Example 3C, the differential drain current ΔI_{ds} are all lower than 0.03µA throughout the first 9 hour of culturing, therefore no blips or sub-blips can be identified in the electrical signal. As shown in FIG. 6H, the number of colony-forming unit (CFU) obtained from agar plating shows a trend of monotonic decreasing, and as shown in FIG. 6I, the pH value of the culturing medium approximately remains unchanged or with a variation less than 0.3 with respect to the initial pH value (t=0), indicating that the electrical signal pattern shown in FIG. 6G corresponds to the biological sample (e.g., Pseudomonas aeruginosa) being sensitive to the interacting agent (e.g., Gentamicin).

### Example 4A

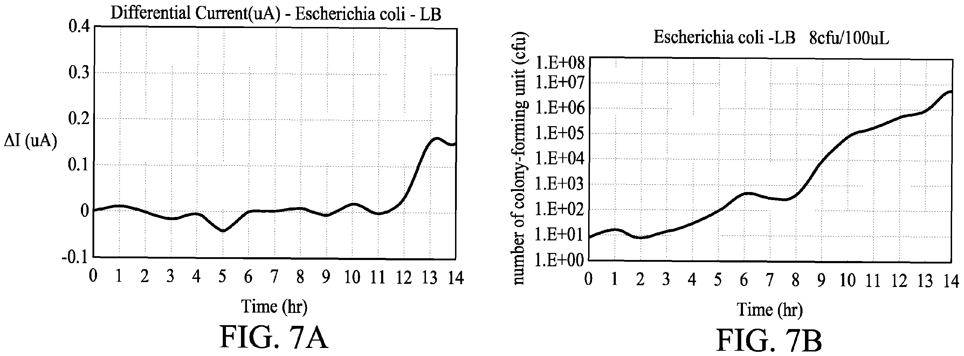

Referring to FIG. 7A and FIG. 7B, FIG. 7A shows a value of current variation (e.g., differential drain current ΔI_{ds}, or equivalent to ΔI_{ds} previously described) with respect to time (Hr) for a biological sample being resistant to a interacting agent, FIG. 7B shows a number of colony-forming unit (CFU) with respect to time (Hr) corresponding to the biological sample of FIG. 7A, in accordance with some embodiments of the present disclosure. Escherichia coli are used as biological sample and no interacting agent is applied in Example 4A.

To prepare the culturing medium, 200 µL of non-buffered LB broth having predetermined dosages (including zero dosage) of interacting agent was inoculated with predetermined concentration of Escherichia coli and cultured at a temperature of 37°C. The sample was subjected to the pathogen detection using the pathogen detection chip and sensor described herein with a front gate voltage of +1.8V and a back gate voltage of -0.5V for drain current I_{ds} measurement at predetermined time points (e.g., at each hour). The differential drain current ΔI_{ds} can be determined using the two approaches previously described, including (1) subtracting the drain current I_{ds} measured from a control group from the drain current I_{ds} measured from an experimental group during the course of culturing; and (2) subtracting the initial drain current I_{ds} (e.g. at t=0) from the drain current I_{ds} measured from an experimental group during the course of culturing. When the former approach is taken, the concentration of biological sample can be determined by agar plating under the same environmental condition.

As shown in FIG. 7A, the differential drain current ΔI_{ds} does not appear to have a local maximum value greater than or equal to 3 times of the base value during the first 11 hours of culturing. As shown in FIG. 7B, the number of colony-forming unit (CFU) obtained from agar plating shows a trend of substantial monotonic increasing after the 2^{nd} hour, indicating that the electrical signal pattern shown in FIG. 7A corresponds to the biological sample (e.g., Escherichia coli) being in an environment free of interacting agent.

### Example 4B

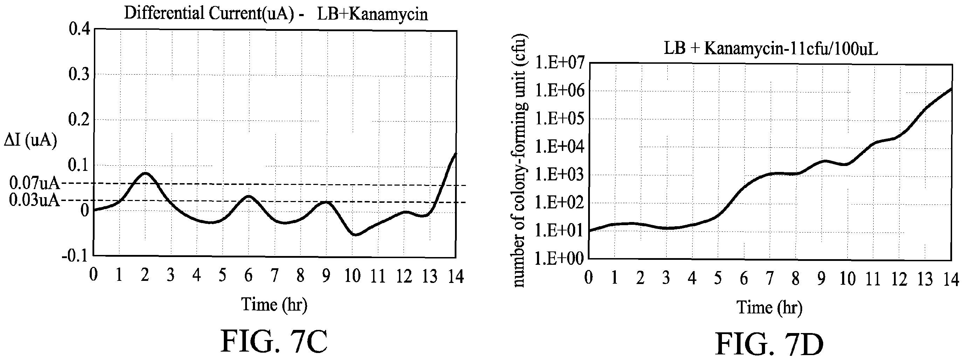

Referring to FIG. 7C and FIG. 7D, FIG. 7C shows a value of current variation (e.g., differential drain current ΔI_{ds}, or equivalent to ΔI_{ds} previously described) with respect to time (Hr) for a biological sample being resistant to a interacting agent, FIG. 7D shows a number of colony-forming unit (CFU) with respect to time (Hr) corresponding to the biological sample of FIG. 7C, in accordance with some embodiments of the present disclosure. Escherichia coli are used as biological sample and Kanamycin is used as an interacting agent in Example 4B.

Sample preparation and measurement condition can be referred to those in Example 4A and are not repeated here for brevity. As shown in FIG. 7C, the differential drain current ΔI_{ds} appear to have a local maximum value greater than or equal to 3 times of the base value at the 2^{nd} hour of culturing. For example, in Example 4B, a differential drain current ΔI_{ds} of greater than 0.07 µA (e.g., about 0.08µA) can be identified as a blip in the electrical signal. In addition, a differential drain current ΔI_{ds} of about 0.04µA can be identified as a sub-blip at the 6^{th} hour. As previously described, electrical signal lower than 0.03µA is not considered as a blip or a sub-blip in some of the embodiments. As a result, a blip at the 2^{nd} hour and a sub-blip at the 6^{th} hour can be identified in FIG. 7C.

As shown in FIG. 7D, the number of colony-forming unit (CFU) obtained from agar plating shows a trend of substantial monotonic increasing after the 3^{rd} hour, indicating that the electrical signal pattern shown in FIG. 7C corresponds to the biological sample (e.g., Escherichia coli) being resistant to the interacting agent (e.g., Kanamycin).

### Example 4C

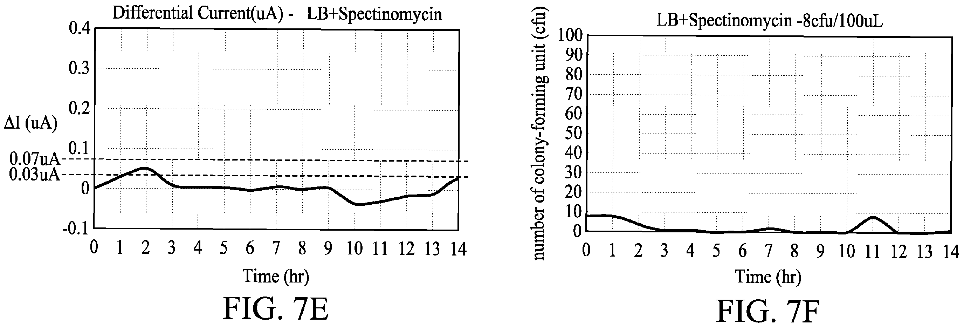

Referring to FIG. 7E and FIG. 7F, FIG. 7E shows a value of current variation (e.g., differential drain current ΔI_{ds}, or equivalent to ΔI_{ds} previously described) with respect to time (Hr) for a biological sample being sensitive to a interacting agent, FIG. 7F shows a number of colony-forming unit (CFU) with respect to time (Hr) corresponding to the biological sample of FIG. 7E, in accordance with some embodiments of the present disclosure. Escherichia coli are used as biological sample and Spectinomycin is used as an interacting agent in Example 4C.

Sample preparation and measurement condition can be referred to those in Example 4A and are not repeated here for brevity. As shown in FIG. 7E, the differential drain current ΔI_{ds} does not appear to have a local maximum value greater than or equal to 3 times of the base value. However, a local maximum can be observed at the 2^{nd} hour of the culturing. For example, in Example 4C, the differential drain current ΔI_{ds} are all lower than 0.07µA throughout the first 9 hour of culturing, therefore no blips can be identified in the electrical signal. The local maximum of about 0.05µA can be identified as a sub-blip in the electrical signal. As shown in FIG. 7F, the number of colony-forming unit (CFU) obtained from agar plating shows a trend of neither increasing or decreasing, indicating that the electrical signal pattern shown in FIG. 7E corresponds to the biological sample (e.g., Escherichia coli) being sensitive to the interacting agent (e.g., Spectinomycin).

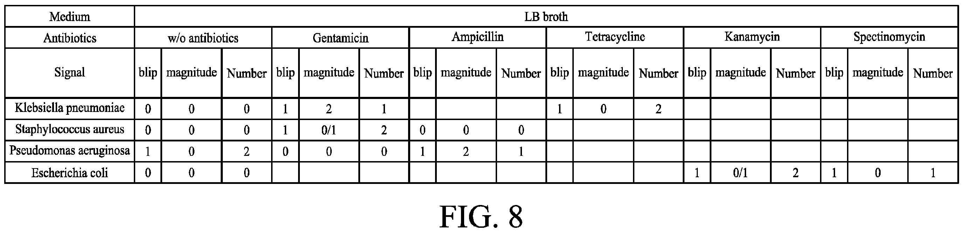

Referring to FIG. 8, FIG. 8 shows a Table summarizing the electrical signal measured in Example 1A to Example 4C. As previously discussed, when taking device-sensitive factor such as background noise into consideration, a differential drain current ΔI_{ds} showing local maximum lower than 0.03µA is not identified as a blip or a sub-blip; a differential drain current ΔI_{ds} showing local maximum greater than 0.03µA and lower than 0.07µA is identified as a sub-blip; a differential drain current ΔI_{ds} showing local maximum greater than 0.07µA is identified as a blip. 0 and 1 indicated in the blip column refer to absence of blip/sub-blip and existence of blip/sub-blip, respectively. 0, 1, and 2 indicated in the magnitude column refer to three levels of electrical signal intensities, including a differential drain current lower than 0.07µA (indicated as "0"), a differential drain current greater than 0.07µA but lower than 0.1µA (indicated as "1 "), and a differential drain current greater than 0.1µA (indicated as "2"). 0, 1, and 2 indicated in the number column refer to the total number of blips and sub-blips.

The reference numbers shown in FIG. 8 may be summarized as Table 1 below.

**Table 1**

| | **Blip** | **magnitude** | **Number** |
|---|---|---|---|
| **0** | absence of blip/sub-blip (current < 0.03µA) | differential drain current lower than 0.07µA | no blip |
| **1** | existence of blip/sub-blip (current > 0.03µA) | differential drain current greater than 0.07µA but lower than 0.1µA | one blip |
| **2** | | differential drain current greater than 0.1µA | two (or more) blips |

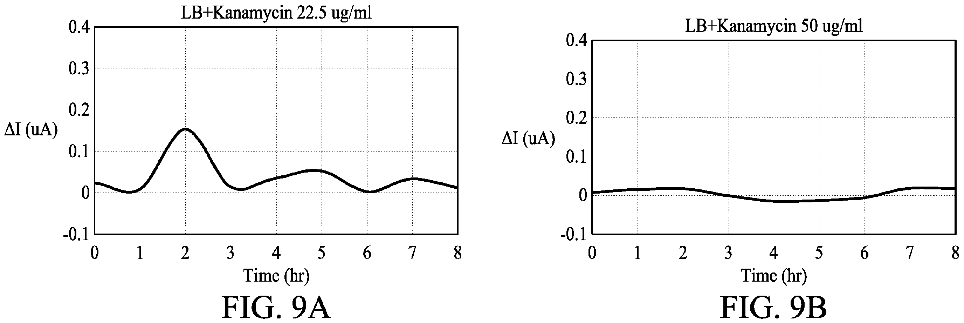

By using the method for pathogen detection described herein, the characteristics of a pathogen (e.g., bacteria) can be extracted from the electrical signal and converted into a series of digits as shown in the Table of FIG. 9. Further information extracted from the electrical signal can be digitized and included in the Table. For example, magnitude of 1 or 2 may indicate the corresponding pathogen being drug resistant to the corresponding interacting agent (e.g., antibiotics). For example, magnitude of 0 may indicate the corresponding pathogen being drug sensitive to the corresponding interacting agent (e.g., antibiotics) or substantially no pathogen.

Furthermore, when an unknown pathogen carried in the body fluid and/or blood of a patient is inoculated in a culturing medium including one or more known interacting agent(s), the digitized information on the look-up Table similar to FIG. 9 can quickly identify the type of pathogen (e.g., within 6 hours), and provide effective treatment to the patient carrying said pathogen.

In addition, the electrical signal recorded with respect to time can provide fingerprint characteristic of the specific pathogen to a specific interacting agent. For example, the electrical signal recorded may correspond to a number of colony-forming unit (CFU) and indicate the pathogen being alive or dead, being active or dormant, being contagious or noncommunicable, or being in one of a growth phases comprising dormant, germination, outgrowth, or vegetative, during the course of culturing.

FIG. 9A and FIG. 9B show electrical signal with respect to time under different dosages of interacting agents, in accordance with some embodiments of the present disclosure. Escherichia coli K12 is used as the pathogen and Kanamycin is used as the interacting agents in FIG. 9A and FIG. 9B. Sample preparation and measurement condition can be referred to those in Example 4A and are not repeated here for brevity. In FIG. 9A, Kanamycin with a dosage of 22.5µg/mL is applied to the culturing medium, and one blip at the 2^{nd} hour of culturing can be identified. The electrical signal indicates that the number of colony-forming unit (CFU) of Escherichia coli K12 still maintain at a certain level at least within several hours of culturing. Alternatively, Kanamycin with a dosage of 22.5µg/mL cannot effectively inhibit the growth of Escherichia coli K12. However, in FIG. 9B, Kanamycin with a dosage of 50µg/mL is applied to the culturing medium, and no blips can be identified during first several hours of culturing. The electrical signal indicates that the number of colony-forming unit (CFU) of Escherichia coli K12 decreases at least within several hours of culturing. Alternatively, Kanamycin with a dosage of 50µg/mL can effectively inhibit the growth of Escherichia coli K12. The transition of the electric signal from the existence of a blip to no blips indicates that a minimum inhibitory concentration (MIC) of Kanamycin with respect to Escherichia coli K12 may sits between the Kanamycin dosages used in the experiment of FIG. 9A and the experiment of FIG. 9B.

Following with a minimum inhibitory concentration (MIC) test for Kanamycin with respect to Escherichia coli K12, Kanamycin with a dosage of 25µg/mL can be determined as the MIC. The dosage of Kanamycin is equivalent to 0.9 time of MIC in the lower dosage experiment of FIG. 9A, therefore the growth of Escherichia coli K12 cannot be effectively inhibited. Whereas the dosage of Kanamycin is equivalent to 2 times of MIC in the higher dosage experiment of FIG. 9B, therefore the growth of Escherichia coli K12 can be effectively inhibited.

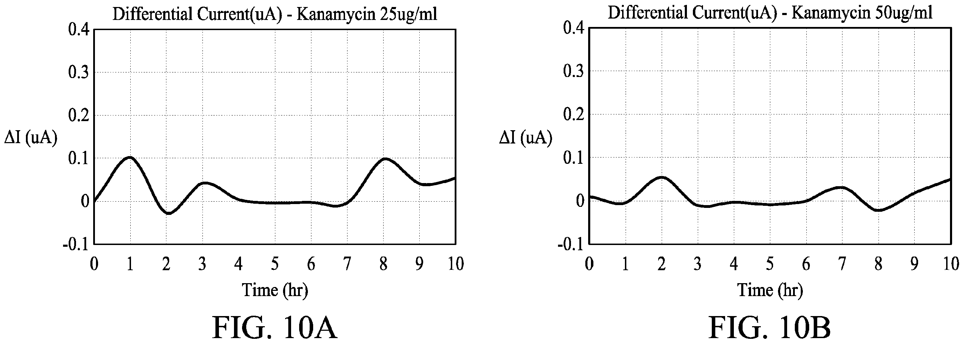

FIG. 10A and FIG. 10B show electrical signal with respect to time under different dosages of interacting agents, in accordance with some embodiments of the present disclosure. Escherichia coli pku57 is used as the pathogen and Kanamycin is used as the interacting agents in FIG. 10A and FIG. 10B. Sample preparation and measurement condition can be referred to those in Example 4A and are not repeated here for brevity. In FIG. 10A, Kanamycin with a dosage of 25µg/mL is applied to the culturing medium, and two blips at the 1^{st} hour and the 8^{th} of culturing can be identified. The electrical signal indicates that the number of colony-forming unit (CFU) of Escherichia coli pku57 still maintain at a certain level at least within several hours of culturing. Alternatively, Kanamycin with a dosage of 25µg/mL cannot effectively inhibit the growth of Escherichia coli pku57. In FIG. 10B, Kanamycin with a dosage of 50µg/mL is applied to the culturing medium, and a blip can be identified at the 2^{nd} hour of culturing. The electrical signal indicates that the number of colony-forming unit (CFU) of Escherichia coli pku57 still maintain at a certain level at least within several hours of culturing. Alternatively, Kanamycin with a dosage of 50µg/mL cannot effectively inhibit the growth of Escherichia coli pku57. Given the consistent showing of at least a blip in the electric signal in both cases, the result indicates that Escherichia coli pku57 behaves drug resistant to Kanamycin at both the lower dosage 25µg/mL used in the experiment of FIG. 10A and the higher dosage 50µg/mL used in the experiment of FIG. 10B since the MIC of Kanamycin with respect to Escherichia coli pku57 is lower than 50µg/mL.

In view of the results in FIG. 9A, FIG. 9B, FIG. 10A, and FIG. 10B, when a blip is identified in the electric signal such as the differential drain current described herein, a monotonic increase spread can be applied to determine whether the pathogen being drug sensitive (e.g., FIG. 9A and FIG. 9B) or drug resistant (e.g., FIG. 10A and FIG. 10B) to the corresponding interacting agent. When a blip cannot be identified in the electric signal pattern, a monotonic decrease spread can be applied to determine whether the pathogen being drug sensitive (e.g., FIG. 9A and FIG. 9B) to the corresponding interacting agent or substantially no pathogen in the culturing medium.

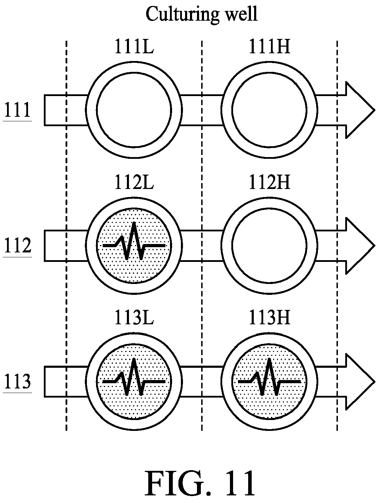

Referring to FIG. 11, FIG. 11 illustrates a plurality of culturing wells or culturing chambers of a pathogen detection chip, each of the three samples are introduced into two culturing wells with differential interacting agent concentration spread, in accordance with some embodiments of the present disclosure. As previously described in FIG. 1, FIG. 2, and FIG. 3, after inoculating the pathogen 207A to the pathogen detection chip 30 through a microfluidic structure 302 connecting to a plurality of individual culturing wells, the pathogen sample is delivered to the designed culturing environment for detection. As shown in FIG. 11, one of the three results can be obtained from the electric signal measurement described herein.

Pathogen sample 111 is inoculated into two culturing wells 111L, 111H. Culturing well 111L includes an interacting agent of a lower dosage, and culturing well 111H includes the same interacting agent of a higher dosage. In some embodiments, the culturing wells 111L may possess an interacting agent dosage lower than the antibiotic sensitive dosage provided by Clinical and Laboratory Standards Institute (CLSI) or lower than MD prescribed dosage. Similarly, the culturing wells 111H may possess an interacting agent dosage greater than the antibiotic sensitive dosage provided by CLSI or greater than MD prescribed dosage. Neither the culturing well 111L nor culturing well 111H shows the electrical signal (i.e., the blip described herein) in first 6 hours of culturing, indicating that pathogen sample 111 can be substantially free of pathogen.

Pathogen sample 112 is inoculated into two culturing wells 112L, 112H. Culturing well 112L includes an interacting agent of a lower dosage, and culturing well 112H includes the same interacting agent of a higher dosage. In some embodiments, the culturing wells 112L may possess an interacting agent dosage lower than the antibiotic sensitive dosage provided by CLSI or lower than MD prescribed dosage. Similarly, the culturing wells 112H may possess an interacting agent dosage greater than the antibiotic sensitive dosage provided by CLSI or greater than MD prescribed dosage. The electrical signal (i.e., the blips described herein) is present in the culturing well 112L but is absent in the culturing well 112H in first 6 hours of culturing. This signal combination not only indicates that pathogen sample 112 includes pathogen but also the pathogen is sensitive to the interacting agent.

Pathogen sample 113 is inoculated into two culturing wells 113L, 113H. Culturing well 113L includes an interacting agent of a lower dosage, and culturing well 113H includes the same interacting agent of a higher dosage. In some embodiments, the culturing wells 113L may possess an interacting agent dosage lower than the antibiotic sensitive dosage provided by CLSI or lower than MD prescribed dosage. Similarly, the culturing wells 113H may possess an interacting agent dosage greater than the antibiotic sensitive dosage provided by CLSI or greater than MD prescribed dosage. The electrical signal (i.e., the blips described herein) is present both in the culturing well 113L and the culturing well 113H in first 6 hours of culturing. This signal combination not only indicates that pathogen sample 113 includes pathogen but also the pathogen is resistant to the interacting agent.

When the interacting agent is to be in a form of energy such as radiation or heat, the culturing well or culturing chamber 111L, 112L, or 113L receives energy of lower intensity, and the culturing well or culturing chamber 111H, 112H, or 113H receives energy of higher intensity that has studied impact to the growth of the pathogen.

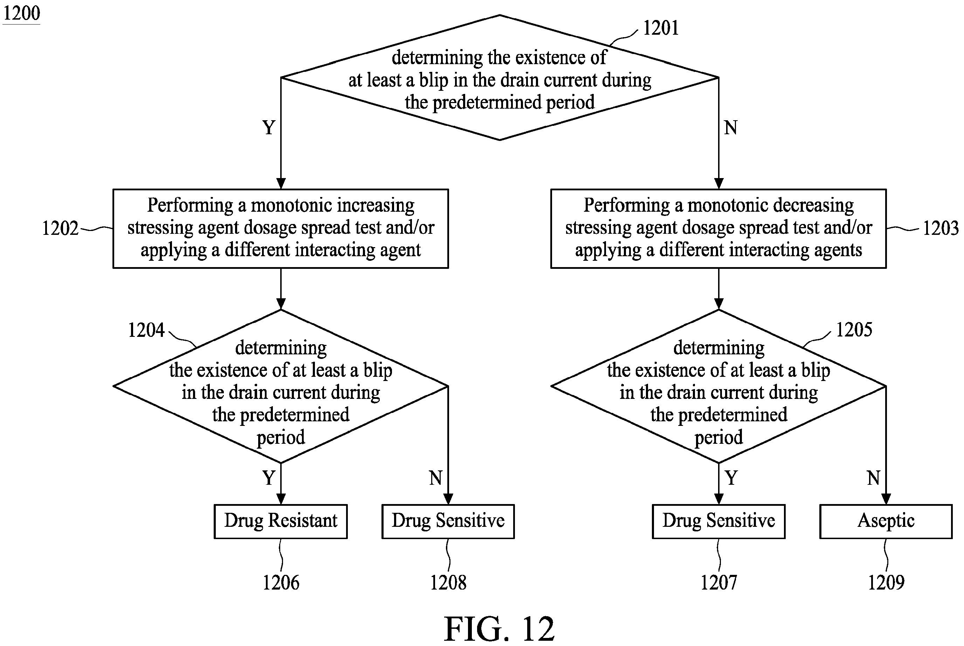

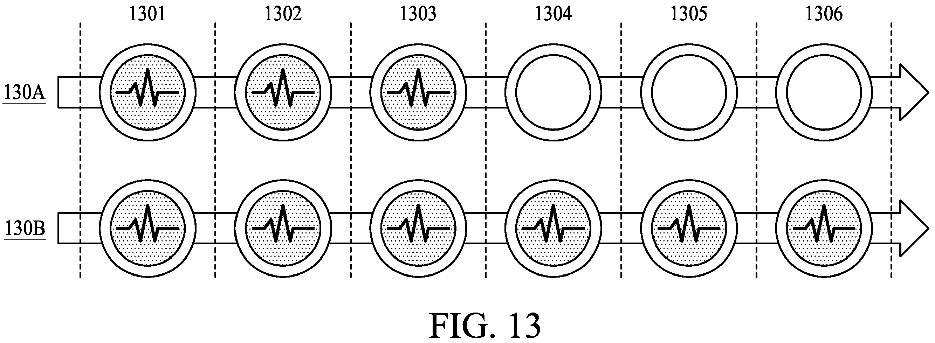

Referring to FIG. 12, FIG. 12 shows a flow chart of a method 1200 for pathogen detection, in accordance with some embodiments of the present disclosure. By carrying out the operations 1201 to 1205 described in the method 1200, the antibiotics susceptibility tests (AST) and the aseptic test can be performed. In operation 1201, the existence of at least one blip in the electrical signal (e.g., the differential drain current) is determined during a predetermined period of culturing (e.g., less than 6 hours). If at least one blip is present in the electrical signal (Y), the flow goes to operation 1202. In operation 1202, a monotonic increasing interacting agent dosage spread test (hereinafter increasing spread test) is performed, or a different interacting agent is applied. Through another operation 1204 of determining the existence of at least a blip all the culturing wells in the increasing spread test, the pathogen in biological sample being resistant 1206 or sensitive 1208 to the interacting agent can be determined. The increasing spread test is further described in FIG. 13.

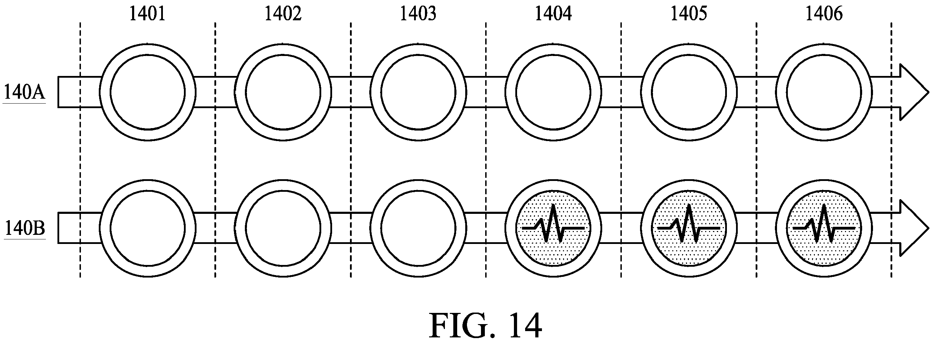

Referring back to operation 1201, the existence of at least one blip in the electrical signal (e.g., the differential drain current) is determined during a predetermined period of culturing (e.g., less than 6 hours). If at least one blip is absent in the electrical signal (N), the flow goes to operation 1203. In operation 1203, a monotonic decreasing interacting agent dosage spread test (hereinafter decreasing spread test) is performed, or a different interacting agent is applied. Through another operation 1205 of determining the existence of at least a blip all the culturing wells in the decreasing spread test, the pathogen in biological sample being sensitive 1207 to the interacting agent or free of pathogen 1209 can be determined. The decreasing spread test is further described in FIG. 14.

Referring to FIG. 13, FIG. 13 illustrates a plurality of culturing wells of a pathogen detection chip, each of the two samples are introduced into six culturing wells with monotonic increasing interacting agent concentration spread, in accordance with some embodiments of the present disclosure. Interacting agent concentration or dosage is monotonically increased from the culturing well 1301 to culturing well 1306, and in some embodiments, the minimum inhibitory concentration (MIC) of the interacting agent is between the lowest concentration and the highest concentration of the spread. The interacting agent concentration in culturing well 1301 coincides with the interacting agent concentration applied in operation 1201. When it is determined that at least a blip is observed in the operation 1201, the pathogen sample previously applied in operation 1201 is inoculated into the culturing well 1301 to 1306. Two conditions 130A, 130B can be expected. Under condition 130A, the presence of electrical signal (e.g., the blips described herein) in the culturing well 1301, 1302, 1303 and the absence of the electrical signal in the culturing well 1304, 1305, 1306 are observed, the result indicates that the pathogen sample is not free of pathogen and that the pathogen is sensitive to the interacting agent. Under condition 130B, the presence of electrical signal (e.g., the blips described herein) in the all six culturing well 1301, 1302, 1303, 1304, 1305, 1306 is observed, the result indicates that the pathogen sample is not free of pathogen and that the pathogen is resistant to the interacting agent.

Referring to FIG. 14, FIG. 14 illustrates a plurality of culturing wells of a pathogen detection chip, each of the two samples are introduced into six culturing wells with monotonic decreasing interacting agent concentration spread, in accordance with some embodiments of the present disclosure. Interacting agent concentration or dosage is monotonically decreased from the culturing well 1401 to culturing well 1406, and in some embodiments, the minimum inhibitory concentration (MIC) of the interacting agent is between the highest concentration and the lowest concentration of the spread. The interacting agent concentration in culturing well 1401 coincides with the interacting agent concentration applied in operation 1201. When it is determined that no blip is observed in the operation 1201, the pathogen sample previously applied in operation 1201 is inoculated into the culturing well 1401 to 1406. Two conditions 140A, 140B can be expected. Under condition 140A, the absence of electrical signal (e.g., the blips described herein) in the all six culturing wells 1401, 1402, 1403, 1404, 1405, 1406 is observed, the result indicates that the pathogen sample is free of pathogen, so as to confirm the aseptic condition of the biological sample. Under condition 140B, the presence of electrical signal (e.g., the blips described herein) in the culturing well 1404, 1405, 1406 and the absence of the electrical signal in the culturing well 1401, 1402, 1403 are observed, the result indicates that the pathogen sample is not free of pathogen and that the pathogen is sensitive to the interacting agent.

Referring to the increasing spread test of FIG. 13 and the decreasing spread test of FIG. 14, in some embodiments, the increasing spread test and the decreasing spread test are designed to encompass the interacting agent concentration greater and lower than the antibiotic sensitive dosage provided by Clinical and Laboratory Standards Institute (CLSI) or MD prescribed dosage, if the MIC of the interacting agent to that particular pathogen is not readily known.

In view of the method 1200 of FIG. 12, the increasing spread test of FIG. 13, and the decreasing spread test of FIG. 14, the pathogen sensing chip described herein can carry out the AST test and aseptic test through several testing operations, each operations may take less than 6 hours of culturing to obtain the electrical signal result. Moreover, since a plurality of culturing wells or culturing chambers may be integrated in the pathogen detection chip described herein, different types of interacting agents and its corresponding increasing or decreasing spread test can be designed in one chip, so as to obtain detection result in a most efficient and cost-saving way.

## Claims

1. A method for pathogen detection, comprising:
applying a biological sample to a culturing chamber comprising an interacting agent, wherein the interacting agent is an agent that interacts with a pathogen; driving a sensing chip in direct contact with a culturing medium containing the interacting agent in the culturing chamber, wherein the culturing medium comprises the biological sample;
measuring an electrical signal from the sensing chip by measuring a drain current of a transistor over a predetermined period, wherein the predetermined period is less than 6 hours;
obtaining pathogen-related information of the biological sample based on the electrical signal; and
determining the existence of at least a blip in the drain current during the predetermined period, wherein the blip is a statistically distinguishable signal in the drain current.

2. The method of claim 1,
wherein the biological sample comprises body fluid, blood, or combinations thereof.

3. The method of at least one of the preceding claims, wherein the applying the biological sample to a plurality of culturing chambers comprises applying the biological sample containing less than 100 colony-forming unit (CFU) per 100 µL.

4. The method of at least one of the preceding claims, wherein the interacting agent comprises an agent causing spore germination, an agent causing oxidative stress, an agent causing chemical damage or enzymatic destruction, an agent causing nutritional deficiency, UV irradiation, bacteriophages, an antibiotics, an agent causing essential ions deficiency, enzymes, radiation, or heat.

5. The method of at least one of the preceding claims, wherein two of the culturing chambers comprise identical interacting agents with different dosages or intensities; or
wherein two of the culturing chambers comprise different interacting agents.

6. The method of at least one of the preceding claims, wherein the pathogen-related information comprises the existence of the pathogen, susceptibility of the pathogen to the interacting agent, dosage of the interacting agent sufficient to induce resistance of the pathogen, dosage of the interacting agent sufficient to suppress activity of the pathogen, and a fingerprint characteristic of the pathogen.

7. The method of claim 6, wherein the fingerprint characteristic of the pathogen comprises the pathogen being alive or dead, being active or dormant, being contagious or noncommunicable, or being in one of phases comprising dormant, germination, outgrowth, vegetative, lag, stationary, or death.

8. The method of at least one of the preceding claims 2 to 7, when at least a blip exists in the current, further comprising performing a monotonic increasing interacting agent dosage spread test with a plurality of culturing wells or applying a different interacting agent to determine whether the pathogen in biological sample being resistant or sensitive to one of the interacting agents.

9. The method of at least one of the preceding claims 2 to 8, when no blip exists in the current, further comprising performing a monotonic decreasing interacting agent dosage spread test with a plurality of culturing wells or applying a different interacting agent to determine whether the biological sample being free of pathogen or pathogen being sensitive to one of the interacting agents.

10. The method of any one of claims 8 or 9, wherein the monotonic increasing interacting agent dosage spread test with a plurality of culturing wells or the monotonic decreasing interacting agent dosage spread test with a plurality of culturing wells each comprises a dosage of minimum inhibitory concentration (MIC) of the one of the interacting agents.

11. The method of claim 1, wherein the blip comprises a local maximum value greater than or equal to 3 times of a base value in the drain current.

12. The method of claim 1, wherein the existence of the blip is determined through a value of current variation with respect to time for the biological sample.

## Patentansprüche

1. Verfahren zum Nachweis von Krankheitserregern, umfassend:
Applizieren einer biologischen Probe in eine Kulturkammer umfassend ein interagierendes Mittel, wobei das interagierende Mittel ein Mittel, welches mit einem Krankheitserreger interagiert, ist, Ansteuern eines Sensorchips in direktem Kontakt mit einem Kulturmedium enthaltend das interagierende Mittel in der Kulturkammer, wobei das Kulturmedium die biologische Probe umfasst;
Messen eines elektrischen Signals von dem Sensorchip durch Messen eines Drainstroms von einem Transistor über eine vorher bestimmte Zeitdauer, wobei die vorher bestimmte Zeitdauer kürzer als 6 Stunden ist,
Erhalten von mit Krankheitserregern in Zusammenhang stehenden Informationen von der biologischen Probe basierend auf dem elektrischen Signal; und
Bestimmen des Vorhandenseins von mindestens einer kurzzeitigen Veränderung in dem Drainstrom während der vorher bestimmten Zeitdauer, wobei die kurzzeitige Veränderung ein statistisch unterscheidbares Signal in dem Drainstrom ist.

2. Verfahren nach Anspruch 1,
wobei die biologische Probe Körperfluid, Blut, oder Kombinationen davon umfasst.

3. Verfahren nach mindestens einem der vorhergehenden Ansprüche, wobei das Applizieren der biologischen Probe in eine Vielzahl von Kulturkammern Applizieren der biologischen Probe enthaltend weniger als 100 koloniebildende Einheiten (CFU) pro 100 µL umfasst.

4. Verfahren nach mindestens einem der vorhergehenden Ansprüche, wobei das interagierende Mittel ein Sporenkeimung verursachendes Mittel, ein oxidativer Stress verursachendes Mittel, ein chemischer Schaden oder enzymatische Zerstörung verursachendes Mittel, ein Nährstoffmangel verursachendes Mittel, UV-Bestrahlung, Bakteriophagen, ein Antibiotikum, ein einen Mangel an essentiellen Ionen verursachendes Mittel, Enzyme, Strahlung, oder Wärme umfasst.

5. Verfahren nach mindestens einem der vorhergehenden Ansprüche, wobei zwei der Kulturkammern identische interagierende Mittel mit unterschiedlichen Dosierungen oder Intensitäten umfassen; oder wobei zwei der Kulturkammern unterschiedliche interagierende Mittel umfassen.

6. Verfahren nach mindestens einem der vorhergehenden Ansprüche, wobei die mit Krankheitserregern in Zusammenhang stehenden Informationen das Vorhandensein von dem Krankheitserreger, die Empfindlichkeit von dem Krankheitserreger in Bezug auf das interagierende Mittel, die Dosierung von dem interagierenden Mittel, welche ausreichend ist, Resistenz von dem Krankheitserreger zu induzieren, die Dosierung von dem interagierenden Mittel, welche ausreichend ist, Aktivität von dem Krankheitserreger zu unterdrücken, und eine Fingerabdruck-Charakteristik von dem Krankheitserreger umfassen.

7. Verfahren nach Anspruch 6, wobei die Fingerabdruck-Charakteristik von dem Krankheitserreger den Krankheitserreger, welcher lebend oder tot ist, welcher aktiv oder nicht aktiv ist, welcher ansteckend oder nicht übertragbar ist, oder welcher in einer der Phasen umfassend nicht aktiv, Keimung, Auswuchs, vegetativ, Lag, stationär, oder Tod ist, umfasst.

8. Verfahren nach mindestens einem der vorhergehenden Ansprüche 2 bis 7, wobei wenn in dem Strom mindestens eine kurzzeitige Veränderung vorhanden ist, ferner umfassend Durchführen eines Dosierungsausweitungstests von gleichbleibend zunehmendem interagierendem Mittel mit einer Vielzahl von Kulturvertiefungen oder Applizieren eines unterschiedlichen interagierenden Mittels, um zu bestimmen, ob der Krankheitserreger in biologischer Probe resistent oder empfindlich in Bezug auf eines der interagierenden Mittel ist.

9. Verfahren nach mindestens einem der vorhergehenden Ansprüche 2 bis 8, wobei wenn in dem Strom keine kurzzeitige Veränderung vorhanden ist, ferner umfassend Durchführen eines Dosierungsausweitungstests von gleichbleibend abnehmendem interagierendem Mittel mit einer Vielzahl von Kulturvertiefungen oder Applizieren eines unterschiedlichen interagierenden Mittels, um zu bestimmen, ob die biologische Probe frei von Krankheitserreger oder der Krankheitserreger empfindlich in Bezug auf eines der interagierenden Mittel ist.

10. Verfahren nach einem der Ansprüche 8 oder 9, wobei der Dosierungsausweitungstest von gleichbleibend zunehmendem interagierendem Mittel mit einer Vielzahl von Kulturvertiefungen oder der Dosierungsausweitungstest von gleichbleibend abnehmendem interagierendem Mittel mit einer Vielzahl von Kulturvertiefungen jeweils eine Dosierung von minimaler inhibitorischer Konzentration (MIC) von dem einen der interagierenden Mittel umfasst.

11. Verfahren nach Anspruch 1, wobei die kurzzeitige Veränderung einen lokalen maximalen Wert von höher als oder gleich dem Dreifachen eines Basiswerts in dem Drainstrom umfasst.

12. Verfahren nach Anspruch 1, wobei das Vorhandensein der kurzzeitigen Veränderung bestimmt wird durch einen Wert von Stromvariation hinsichtlich Zeit für die biologische Probe.

## Revendications

1. Procédé de détection d'agent pathogène, comprenant :
l'application d'un échantillon biologique à une chambre de culture comprenant un agent d'interaction, dans lequel l'agent d'interaction est un agent qui interagit avec un agent pathogène ; la mise en contact direct d'une puce de détection avec un milieu de culture contenant l'agent d'interaction dans la chambre de culture, dans lequel le milieu de culture comprend l'échantillon biologique ;
la mesure d'un signal électrique provenant de la puce de détection en mesurant un courant de drain d'un transistor sur une période prédéterminée, dans lequel la période prédéterminée est inférieure à 6 heures ;
l'obtention d'informations relatives à un agent pathogène de l'échantillon biologique sur la base du signal électrique ; et
la détermination de l'existence d'au moins une anomalie dans le courant de drain pendant la période prédéterminée, dans lequel l'anomalie est un signal statistiquement distinct dans le courant de drain.

2. Procédé selon la revendication 1,
dans lequel l'échantillon biologique comprenant un fluide corporel, du sang ou des combinaisons de ceux-ci.

3. Procédé selon au moins l'une des revendications précédentes, dans lequel l'application de l'échantillon biologique à une pluralité de chambres de culture comprend l'application de l'échantillon biologique contenant moins de 100 unités formant colonie (UFC) pour 100 µL.

4. Procédé selon au moins l'une des revendications précédentes, dans lequel l'agent d'interaction comprend un agent provoquant la germination de spores, un agent provoquant un stress oxydatif, un agent provoquant des dommages chimiques ou une destruction enzymatique, un agent provoquant une carence nutritionnelle, un rayonnement UV, des bactériophages, un antibiotique, un agent provoquant une carence en ions essentiels, des enzymes, des radiations ou de la chaleur.

5. Procédé selon au moins l'une des revendications précédentes, dans lequel deux des chambres de culture comprennent des agents d'interaction identiques avec des dosages ou des intensités différents ; ou
dans lequel deux des chambres de culture comprennent des agents d'interaction différents.

6. Procédé selon au moins l'une des revendications précédentes, dans lequel les informations relatives à l'agent pathogène comprennent l'existence de l'agent pathogène, la susceptibilité de l'agent pathogène à l'agent d'interaction, le dosage de l'agent d'interaction suffisant pour induire une résistance de l'agent pathogène, le dosage de l'agent d'interaction suffisant pour supprimer l'activité de l'agent pathogène, et une caractéristique d'empreinte digitale de l'agent pathogène.

7. Procédé selon la revendication 6, dans lequel la caractéristique d'empreinte digitale de l'agent pathogène comprend le fait que l'agent pathogène est vivant ou mort, est actif ou dormant, est contagieux ou non transmissible, ou est dans l'une des phases comprenant dormant, germination, excroissance, végétatif, latent, stationnaire, ou mort.

8. Procédé selon au moins l'une des revendications 2 à 7 précédentes, lorsqu'au moins une anomalie existe dans le courant, comprenant en outre la réalisation d'un test d'étalement de dosage d'agent d'interaction à croissance monotone avec une pluralité de puits de culture ou l'application d'un agent d'interaction différent pour déterminer si l'agent pathogène présent dans l'échantillon biologique est résistant ou sensible à l'un des agents d'interaction.

9. Procédé selon au moins l'une des revendications 2 à 8 précédentes, lorsque aucune anomalie n'existe dans le courant, comprenant en outre la réalisation d'un test d'étalement de dosage d'agent d'interaction à décroissance monotone avec une pluralité de puits de culture ou l'application d'un agent d'interaction différent pour déterminer si l'échantillon biologique est exempt d'agent pathogène ou si l'agent pathogène est sensible à l'un des agents d'interaction.

10. Procédé selon l'une quelconque des revendications 8 ou 9, dans lequel le test d'étalement de dosage d'agent d'interaction à croissance monotone avec une pluralité de puits de culture ou le test d'étalement de dosage d'agent d'interaction à décroissance monotone avec une pluralité de puits de culture comprennent chacun un dosage de concentration inhibitrice minimale (MIC) de l'agent d'interaction parmi les agents d'interaction.

11. Procédé selon la revendication 1, dans lequel l'anomalie comprend une valeur maximale locale supérieure ou égale à 3 fois une valeur de base dans le courant de drain.

12. Procédé selon la revendication 1, dans lequel l'existence de l'anomalie est déterminée par une valeur de variation de courant par rapport au temps pour l'échantillon biologique.
